(19) 

Europäisches
Patentamt
European
Patent Office
Office européen
des brevets

(11) **EP 3 315 514 B1**

(12) **EUROPEAN PATENT SPECIFICATION**

(45) Date of publication and mention
of the grant of the patent:
**18.06.2025 Bulletin 2025/25**

(21) Application number: **17199410.6**

(22) Date of filing: **26.08.2013**

(51) International Patent Classification (IPC):
**C07K 16/28** (2006.01)   **C07K 16/18** (2006.01)
**C07K 16/46** (2006.01)   **A61K 39/395** (2006.01)
**A61P 25/28** (2006.01)

(52) Cooperative Patent Classification (CPC):
**C07K 16/18; A61P 25/00; A61P 25/28;**
**A61P 43/00; C07K 16/2881;** A61K 2039/505;
C07K 2317/21; C07K 2317/31; C07K 2317/34;
C07K 2317/35; C07K 2317/55; C07K 2317/64;
C07K 2317/732; C07K 2319/33

(54) **BLOOD BRAIN BARRIER SHUTTLE**

BLUT-HIRN-SCHRANKEN-SHUTTLE

NAVETTE DE LA BARRIÈRE HÉMATO-ENCÉPHALIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.08.2012 EP 12182181**

(43) Date of publication of application:
**02.05.2018 Bulletin 2018/18**

(62) Document number(s) of the earlier application(s) in
accordance with Art. 76 EPC:
**13756076.9 / 2 890 712**

(73) Proprietor: **F. Hoffmann-La Roche AG**
**4070 Basel (CH)**

(72) Inventors:
• **BOHRMANN, Bernd**
**4125 Riehen (CH)**
• **FRESKGARD, Per-Ola**
**4153 Reinach BL (CH)**
• **MAIER, Peter**
**88400 Biberach an der Riss (DE)**
• **NIEWOEHNER, Jens**
**81476 München (DE)**
• **TISSOT-DAGUETTE, Alain**
**82061 Neuried (DE)**
• **URICH, Eduard**
**4053 Basel (CH)**

(74) Representative: **Küng, Peter**
**F. Hoffmann-La Roche AG**
**Patent Department**
**Grenzacherstrasse 124**
**4070 Basel (CH)**

(56) References cited:
WO-A1-2007/068429     WO-A1-2012/075037
US-A1- 2005 147 613    US-A1- 2010 081 796
US-A1- 2010 256 338

• **KONTERMANN ROLAND E: "Dual targeting
strategies with bispecific antibodies", MABS,
vol. 4, no. 2, March 2012 (2012-03-01), pages 182 -
197, XP002698995**
• **FELIX WEBER ET AL: "Brain Shuttle Antibody for
Alzheimer's Disease with Attenuated Peripheral
Effector Function due to an Inverted Binding
Mode", CELL REPORTS, vol. 22, no. 1, 2 January
2018 (2018-01-02), US, pages 149 - 162,
XP055578810, ISSN: 2211-1247, DOI: 10.1016/
j.celrep.2017.12.019**
• **J. A. COUCH ET AL: "Addressing Safety
Liabilities of TfR Bispecific Antibodies That
Cross the Blood-Brain Barrier", SCIENCE
TRANSLATIONAL MEDICINE, vol. 5, no. 183, 1
May 2013 (2013-05-01), US, pages 183ra57 -
183ra57, XP055531363, ISSN: 1946-6234, DOI:
10.1126/scitranslmed.3005338**

**(Cont. next page)**

Remarks:

The complete document including Reference
Table(s) and the Sequence Listing(s) can be
downloaded from the EPO website

## Description

The present invention relates to a blood brain barrier shuttle that binds receptors on the blood brain barrier (R/BBB) and methods of using the same.

BACKGROUND

Brain penetration of neurological disorder drugs such as e.g. large biotherapeutic drugs or small molecule drugs having a low brain penetration, is strictly limited by the extensive and impermeable blood-brain barrier (BBB) together with the other cell component in the neurovascular unit (NVU). Many strategies to overcome this obstacle have been tested and one is to utilize transcytosis pathways mediated by endogenous receptors expressed on the brain capillary endothelium. Recombinant proteins such as monoclonal antibodies or peptides have been designed against these receptors to enable receptor-mediated delivery of biotherapeutics to the brain. However, strategies to maximize brain uptake while minimizing miss-sorting within the brain endothelial cells (BECs), and the extent of accumulation within certain organelles (especially organelles that leads to degradation of the biotherapeutic) in BECs, remain unexplored.

Monoclonal antibodies and other biotherapeutics have huge therapeutic potential for treatment of pathology in the central nervous system (CNS). However, their route into the brain is prevented by BBB. Previous studies have illustrated that a very small percentage (approximately 0.1%) of an IgG injected in the bloodstream are able to penetrate into the CNS compartment (Felgenhauer, Klin. Wschr. 52: 1158-1164 (1974)). This will certainly limit any pharmacological effect due to the low concentration within CNS of the antibody.

Therefore, there is a need for delivery systems of neurological disorder drugs across the BBB to shuttle the drugs into the brain efficiently.

WO 2012/075037 describes bispecific anti-TfR x anti-Abeta and bispecific anti-TfR x anti-BACE1 antibodies with a IgG heterotetrameric format wherein one arm binds TfR and the other arm binds the brain target, which function as blood brain barrier shuttle.

SUMMARY

The present invention provides a blood brain barrier shuttle comprising a full length IgG antibody directed to a brain antigen, a linker and one scFab directed to the transferrin receptor, wherein the scFab is coupled by the linker to the C-terminal end of the Fc part of one of the heavy chains of the full length IgG antibody

In a particular embodiment of the blood brain barrier shuttle, the scFab recognizes an epitope in the transferrin receptor comprised within the amino acid sequence of Seq. Id. No. 14, 15 or 16.

In a particular embodiment of the blood brain barrier shuttle, the full length IgG antibody directed to a brain target is a full length IgG antibody directed to Aβ.

In a particular embodiment of the blood brain barrier shuttle, the full length IgG antibody directed to Aβ comprises (a) H-CDR1 comprising the amino acid sequence of Seq. Id. No. 5, (b) H-CDR2 comprising the amino acid sequence of Seq. Id. No. 6, (c) H-CDR3 comprising the amino acid sequence of Seq. Id. No. 7, (d) L-CDR1 comprising the amino acid sequence of Seq. Id. No. 8, (e) L-CDR2 comprising the amino acid sequence of Seq. Id. No. 9 and (f) L-CDR3 comprising the amino acid sequence of Seq. Id. No. 10.

In a particular embodiment of the blood brain barrier shuttle, the full length IgG antibody directed to Abeta comprises a $V_H$ domain comprising the amino acid sequence of Seq. Id. No. 11 and a $V_L$ domain comprising the amino acid sequence of Seq. Id. No. 12.

In a particular embodiment of the blood brain barrier shuttle, the full length IgG antibody directed to a brain target is a full length IgG antibody directed to phosphorylated Tau.

In a particular embodiment of the blood brain barrier shuttle, the full length IgG antibody directed to a brain target is a full length IgG antibody directed to alpha synuclein.

In a particular embodiment of the blood brain barrier shuttle, the linker is a peptide linker, preferably a peptide which is an amino acid sequence with a length of at least 20 amino acids, more preferably with a length of 25 to 50 amino acids.

BRIEF DESCRIPTION OF THE FIGURES

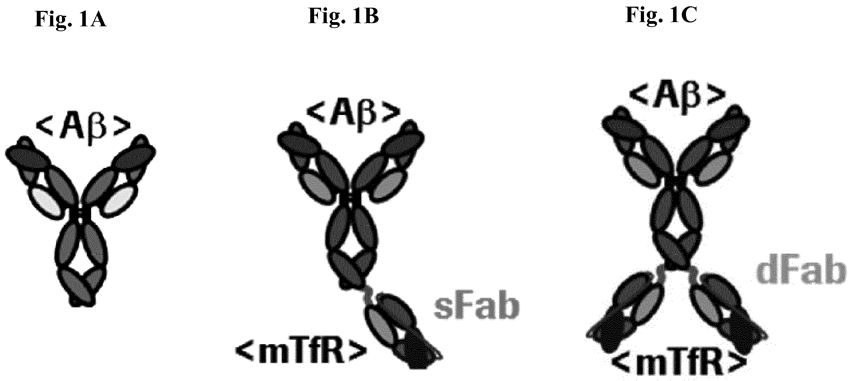

Fig. 1: Different format of blood brain barrier shuttles (fusion proteins) used in the examples. 1A: IgG directed to Aβ (mAb31). 1B: single Fab (scFab) directed to TfR coupled to the Fc part of an IgG directed to Aβ (mAb31). 1C: double Fab (dFab) directed to TfR coupled to the Fc part of an IgG directed to Aβ (mAb31). The scFab structure is fused to the C-terminal end of the heavy chain of the IgG antibody.

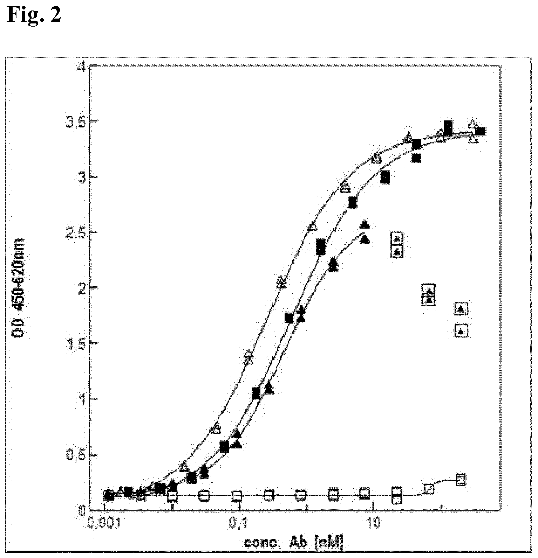

Fig. 2: Binding properties of the fusion proteins towards Aβ structures. The binding affinity was measured using an ELISA setup which shows that the Fab constructs have preserved Aβ binding properties. Binding of mAb31-8D3 constructs to Abeta fibrils. While 8D3 (open squares) does not bind to immobilized Abeta fibrils, mAb31-8D3-dFab (filled squares), mAb31-8D3-sFab (open triangles) and mAb31 (filled triangles) bind with comparable affinities.

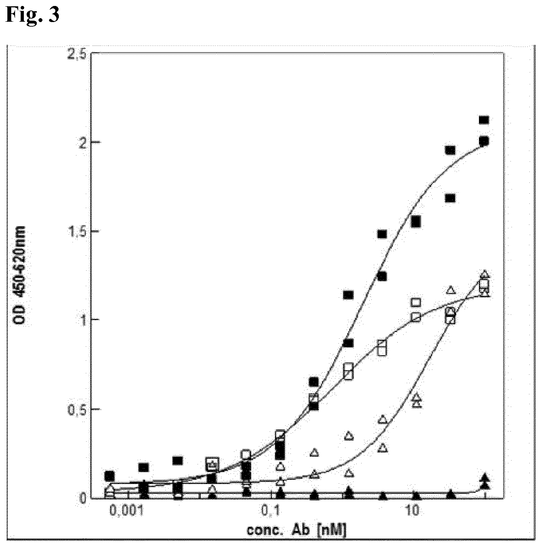

Fig. 3: Binding properties of the constructs towards the Transferrin receptor (TfR). The binding affinity was measured using an ELISA setup which shows that only the Fab constructs binds the Transferrin receptor (TfR) and the double Fab construct have slightly higher apparent affinity due to the bivalent binding mode. Binding of mAb31-8D3 constructs to mTfR. While mAb31 (filled triangles) does not bind to immobilized mTfR, mAb31-8D3-dFab (filled squares) binds with an affinity comparable to that of the 8D3 parent antibody (open squares). The monovalent construct mAb31-8D3-sFab (open triangles) shows an intermediate binding affinity.

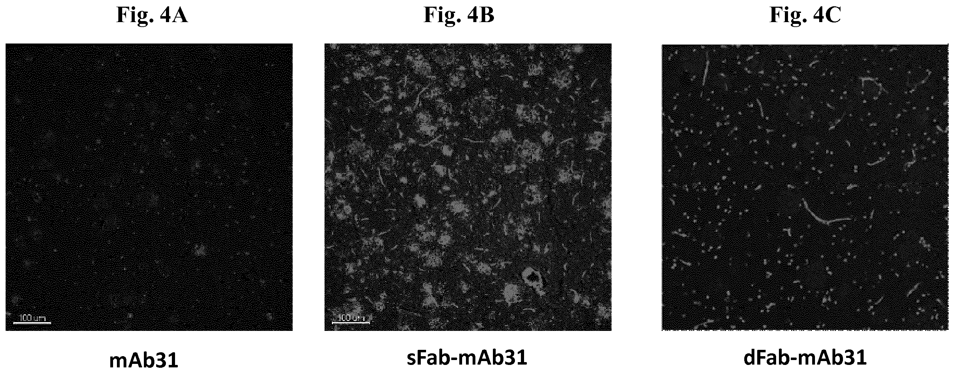

Fig. 4: Plaque decoration of anti-Aβ monoclonal antibody mAb31 (Fig. 4A), single Fab mAb31 (single Fab fused to the C-terminal end of mAb31) (Fig. 4B) and double Fab mAb31 (double Fabs fused to the C-terminal end of mAb31) (Fig. 4C). Construct injected in PS2APP mice (n=3/construct), single intravenous dose 10 mg/kg and then brain perfusion 8 hours post dose. Analysis included immunohistochemistry and confocal microscopy for plaque binding. Data shows that only the single Fab-mAb31 construct are able to cross the BBB and bind to the plaques. The figure shows one representative area of the brain from all animals.

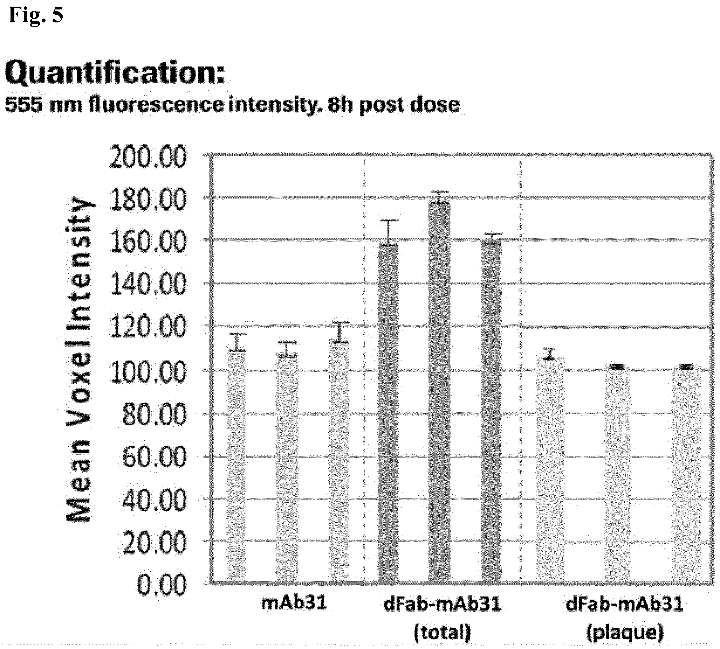

Fig. 5: Shows the quantification of the double Fab-mAb31 construct. The plaque and capillary staining was quantified in all three treated animals in three different regions (9 areas in total for each construct). The data shows that there is only an increase in the capillaries for the double Fab-mAb31 construct compared to mAb31. No increased levels of the double Fab-mAb31 at the plaque (inside the brain) were detected. Quantification of mab31 (HEK control) vs double Fab-mab3, 10 mg/kg, 8h post dose.

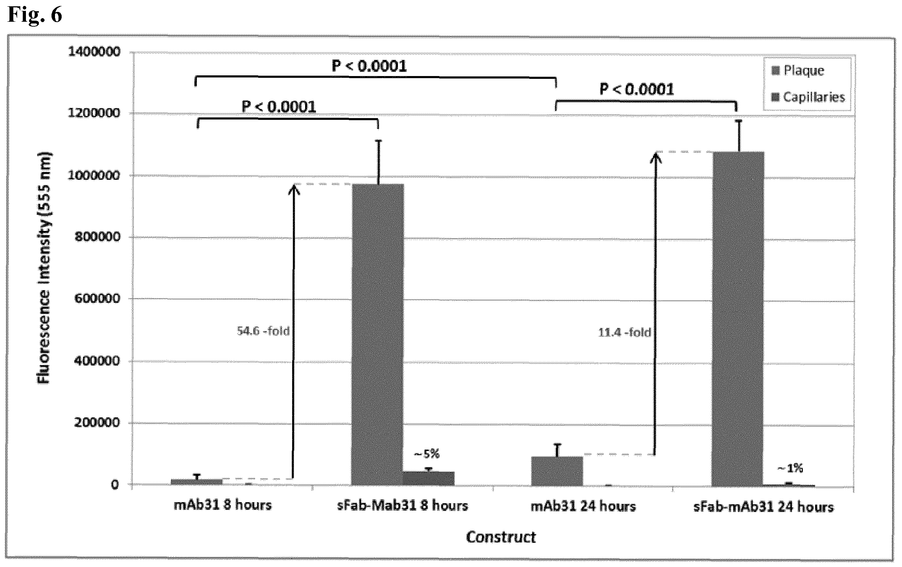

Fig. 6: Shows the quantification of the single Fab-mAb31 construct. The plaque and capillary staining was quantified in all three treated animals in three different regions (9 areas in total for each construct). The data shows that there is a massive increase at the plaques for the single Fab-mAb31 construct compare to mAb31. Quantification of the fluorescence signal indicates more that 50-fold increase of the single Fab-mAb31 compare to the mAb31 construct. There is also a transient staining in the capillaries for the single Fab-mAb31 construct compare to mAb31 indicating the crossing over the BBB. Quantification of mab31 (HEK control) vs single Fab-mab31 10 mg/kg, 8h post dose and 25 mg/kg, 24h post dose.

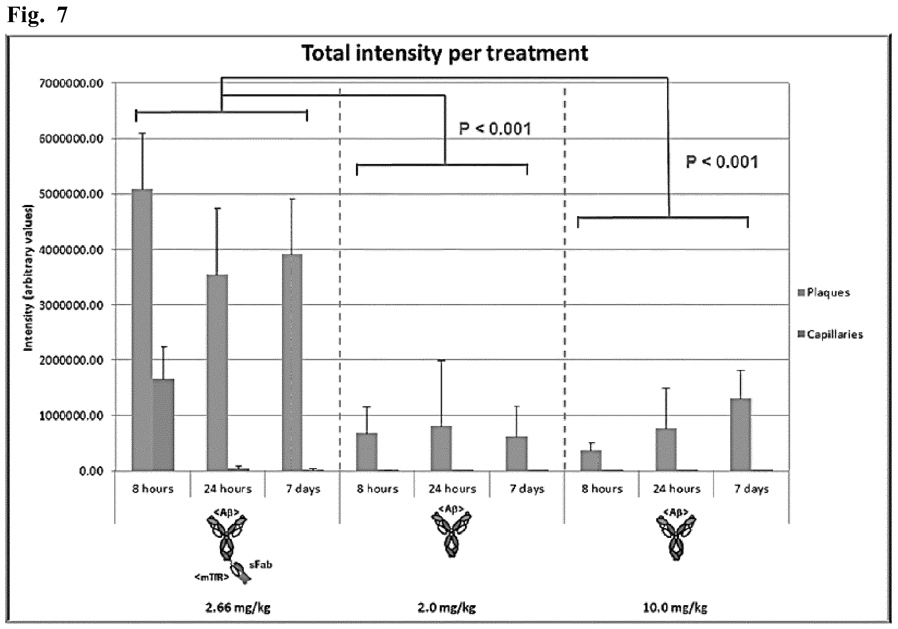

Fig. 7: Plaque decoration of anti-Aβ monoclonal antibody mAb31 at two different doses and single Fab mAb31 (single Fab fused to the C-terminal end of mAb31) at a very low dose. Construct injected in PS2APP mice (n=3/construct), single intravenous dose and then brain perfusion at various time points post dose. Analysis included immunohistochemistry and confocal microscopy for plaque binding. Data shows that only the single Fab-mAb31 construct are able to cross the BBB and bind to the plaques. The brain exposure is very rapid for the single Fab-mAb31 construct and the plaque decoration is sustainable over at least one week from a single administration.

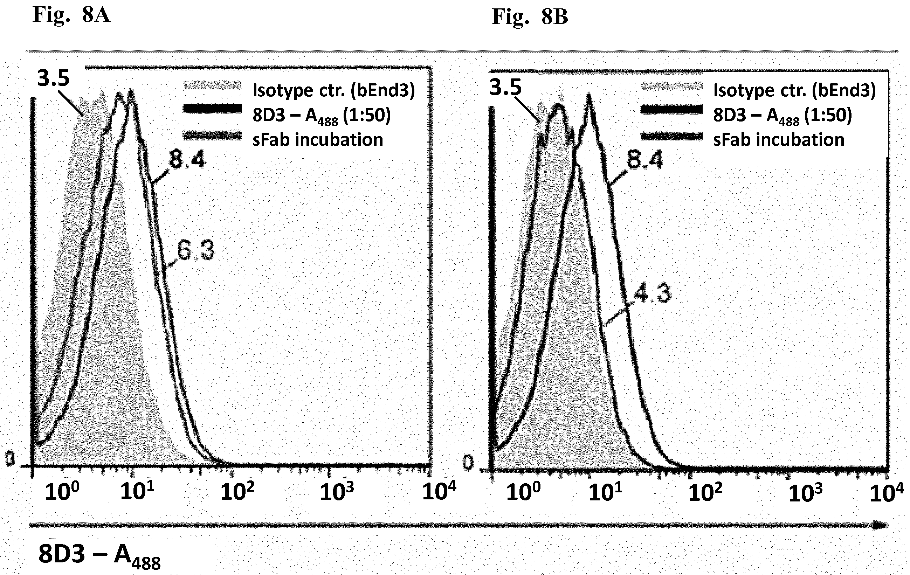

Fig. 8: Quantification of cell surface expression of TfR treated with single Fab-mab31 or double Fab-mAb31. Transferrin receptor (TfR) cell surface down-regulation by the double Fab-mAb31 construct. Brain endothelial cells expressing the TfR were incubated for 24 hours with either the single Fab-mAb31 construct (Fig. 8A) or the double Fab-mAb31 construct (Fig. 8B). Only the double Fab-mAb31 construct lowered the level of cell surface expressed TfR.

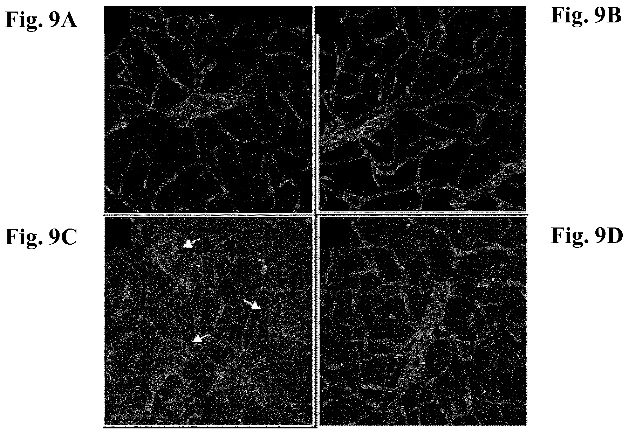

Fig. 9: In vivo cell trafficking of TfR treated with single Fab-mab31 or double Fab-mAb31. Early time points investigating capillary and plaque staining in vivo. Both sFab-MAb31 (Fig. 9A) and dFab-MAb31 (Fig. 9B) decorates the brain vasculature 15 minutes after injection with no difference in their distribution. 8 hours post-injection, sFab-MAb31 reaches the parenchyma and decorates amyloid plaques (arrows, Fig 9C) whereas dFab-MAb31 stays within brain vasculature similarly to the 15 minutes time point (Fig. 9D). No amyloid plaques in the parenchyma are detected for the dFab-MAb31.

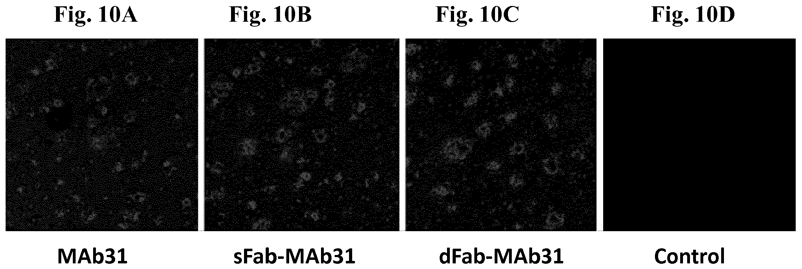

Fig. 10: In vivo cell trafficking of TfR treated with single Fab-mab31 or double Fab-mAb31. To control the integrity of all constructs used in the study, staining of 18 months mouse APP transgenic brain cryosections was done using MAb31 (Fig. 10A), sFab-MAb31 (Fig. 10B) or dFab-MAB31 (Fig. 10C). Fig. 10D shows the results of the control. Results showed that all 3 constructs detected amyloid plaques in the brain of transgenic mice.

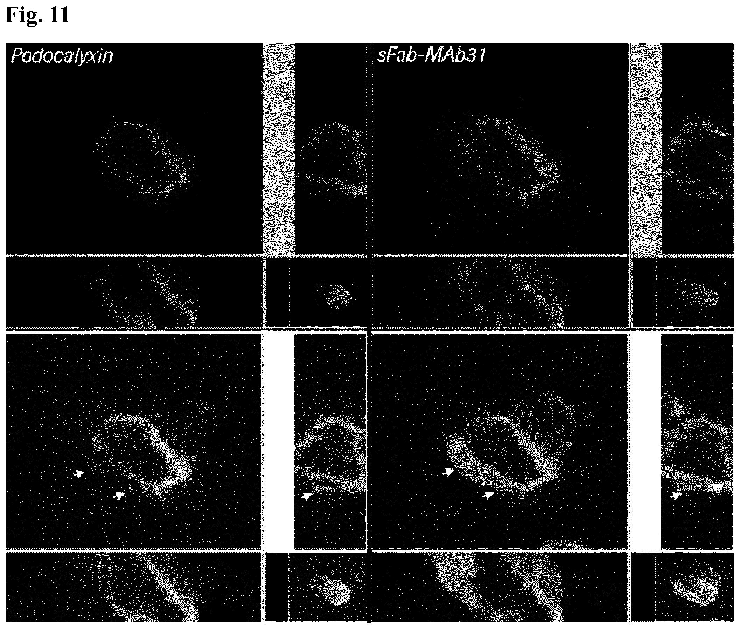

Fig. 11: In vivo cell trafficking of TfR treated with single Fab-mab31. High resolution confocal microscopy on in vivo treated samples shows that sFab-MAb31 do not decorate the luminal side of brain capillaries but are contained within vesicle-like structures crossing the luminal membrane of endothelial cells and within the endothelial cell cytosol. Arrows in Fig 11 indicate vesicles containing sFab-MAb31 constructs on the abluminal side of endothelial cell nuclei. These data suggest that both sFab-MAb31 can enter the brain endothelial cells and cross the vasculature and reach amyloid plaques within the parenchyma space of the brain (Compare with Figure 9A and C).

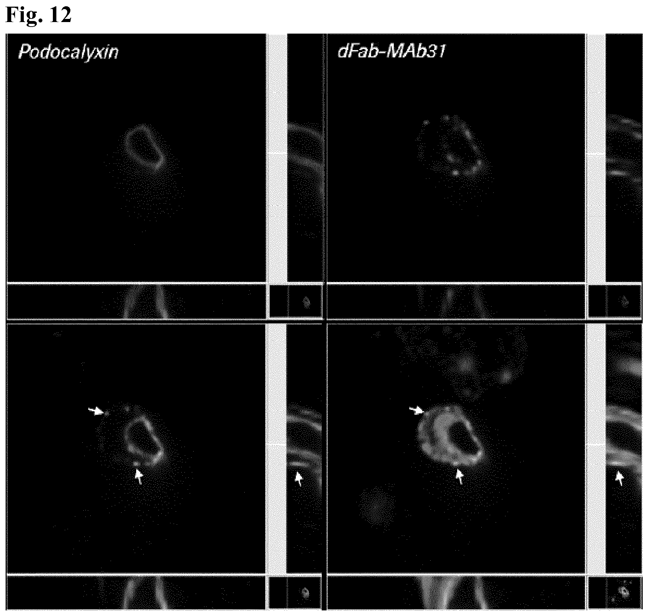

Fig. 12: In vivo cell trafficking of TfR treated with double Fab-mab31. High resolution confocal microscopy on in vivo treated samples shows dFab-MAb31 do not decorate the luminal side of brain capillaries but are contained within vesicle-like structures crossing the luminal membrane of endothelial cells and within the endothelial cell cytosol. Arrows in Fig 12 indicate vesicles containing dFab-MAb31 constructs on the abluminal side of endothelial cell nuclei. These data suggest that dFab-MAb31 can enter the brain endothelial cells but are trapped not able to cross the vasculature and therefore not reach the amyloid plaques within the parenchyma space of the brain (Compare with Figure 9B and D).

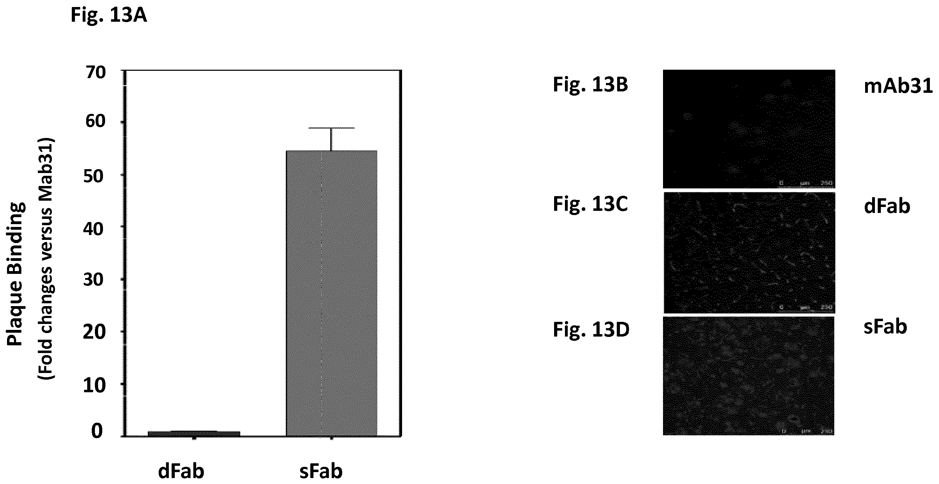

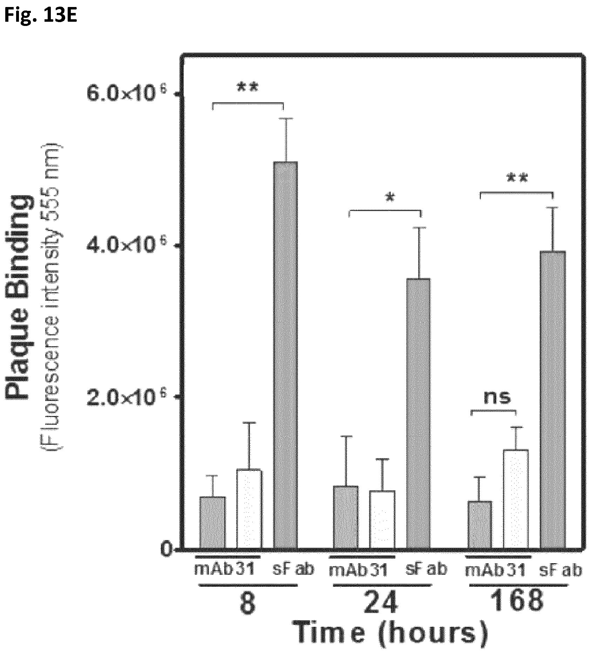

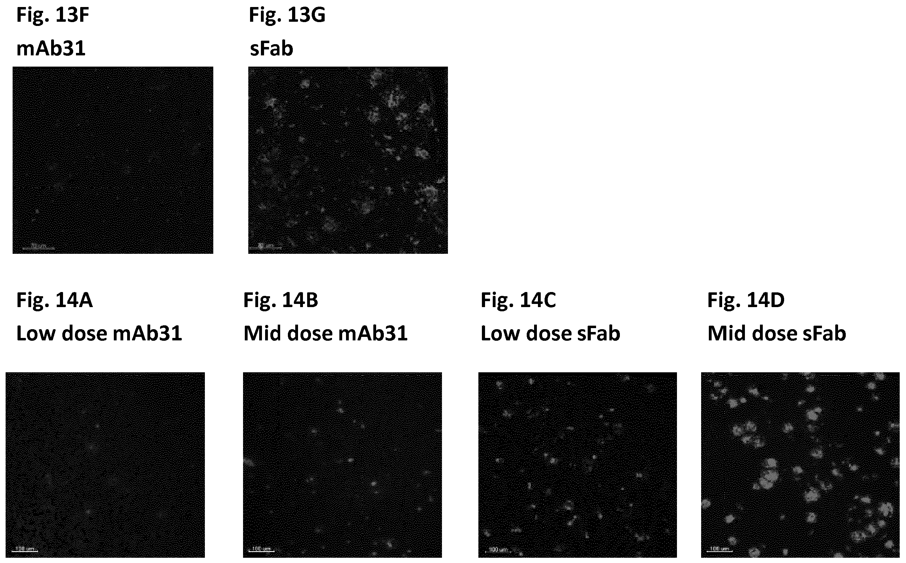

Fig. 13: Brain exposure and plaque decoration after i.v. administration. Fig. 13A: mAb31, dFab and scFab constructs were intravenously injected in PS2APP transgenic animals at 10 mg/kg, animals were perfused and sacrificed 8 hours post injection. No significant increase in plaque decoration was detected for the dFab compared to mAb31. For the sFab construct a 55-fold higher plaque decoration was detected than the parent mAb31 based on fluorescence intensity at 555 nm from the detection antibody. Representative immunohistochemistry staining in cortex of mAb31 (Fig. 13B), dFab (Fig. 13C) and sFab (Fig. 13D) 8 hour post injection. The dFab shows only microvessel staining while the sFab decorates the amyloid-$\beta$ plaques extensively. Fig. 13E: Demonstration that a low dose of the sFab construct (2.66 mg/kg) rapidly and significantly reaches the plaques in the brain compared to both 2 mg/kg and 10 mg/kg of mAb31. The target engagement of the sFab construct is sustainable over at least one week post injection. Immunohistochemistry staining shows plaque decoration for mAb31 at 2 mg/kg (Fig. 13F) and sFab at 2.66 mg/kg (Fig. 13G) 7 days post injection.

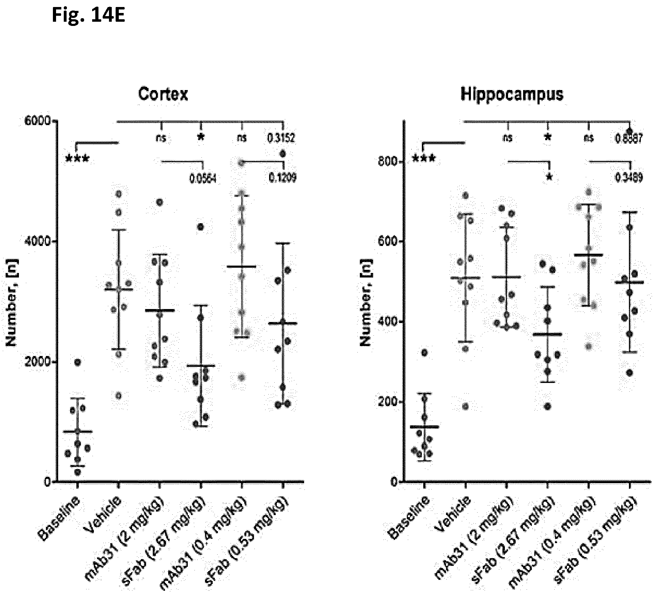

Fig. 14: In vivo efficacy in a chronic study in plaque bearing PS2APP mice treated by 14 weekly i.v. injections. Target plaque binding of administrated constructs bound to residual plaques at the end of the study are shown for low dose mAb31, mid dose mAb31, low dose sFab and mid dose sFab, respectively (Fig. 14A-D). Quantitative morphometric analysis after immunohistochemical staining of plaques is shown for cortex and hippocampus (Fig. 14 E). Plaque load of untreated animals sacrificed at an age of 4.5 months is shown as baseline level of amyloidosis at the start of the study. A significant reduction in plaque numbers is evident after treatment with mid dose sFab compared to the progressive plaque formation seen in vehicle treated animals; a trend of reduced plaque formation appears even at the low dose sFab. Thus, sFab construct significantly reduces plaque numbers in both cortex and hippocampus. Analysis of plaque sizes revealed reduction of plaque numbers most pronounced for small plaque sizes. *p$\leq$0.05, **p$\leq$0.01, ***p$\leq$0.001.

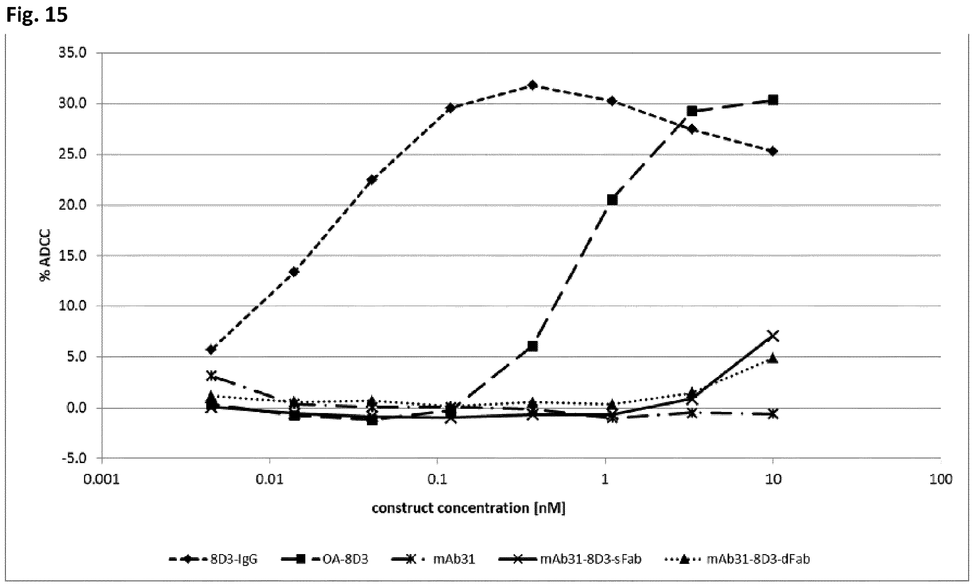

Fig. 15: Antibody multimeric with TfR scFab fragments fused to the Fc C-terminus do not induce ADCC. NK92-mediated killing of BA/F3 mouse erythroleukemia cells was measured by quantifying LDH release. Only multimeric constructs with the TfR-binding Fab moiety in the "conventional" "N-terminal to Fc" orientation induce significant ADCC, while the brain shuttle constructs in reverse orientation are silent.

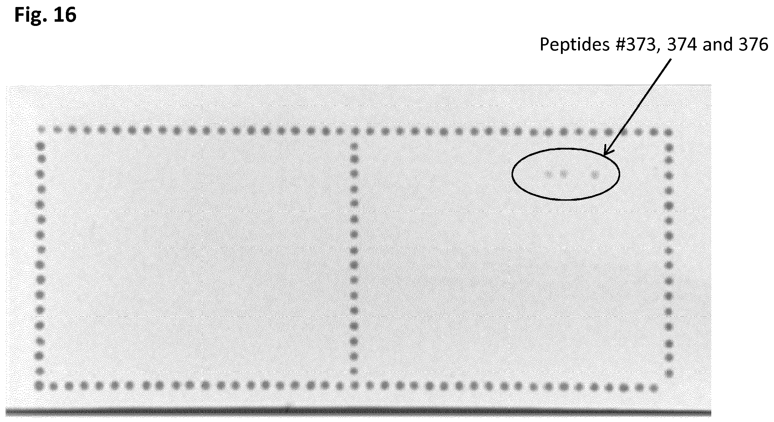

Fig. 16: scFab 8D3 directed to the transferrin receptor binds to three distinct peptides in the extracellular domain of mouse transferrin receptor. Binding of antibody 8D3 to 15mer peptides overlapping by three amino acids was revealed by chemiluminescent detection of antibody incubated on a CelluSpot slide carrying immobilized mTfR peptides. Box: Peptides #373, 374 and 376 bound by 8D3 (Seq. Id. No. 15, 16 and 17).

## DETAILED DESCRIPTION OF EMBODIMENTS OF THE INVENTION

The invention is defined by the appended claims. The technical information set out below may in some respects go beyond the scope of the invention, which is defined exclusively by the appended claims. Any additional technical information which does not fall within the scope of the appended claims, is not part of the invention.

DEFINITIONS

The "blood-brain barrier" or "BBB" refers to the physiological barrier between the peripheral circulation and the brain and spinal cord which is formed by tight junctions within the brain capillary endothelial plasma membranes, creating a tight barrier that restricts the transport of molecules into the brain, even very small molecules such as urea (60 Daltons). The BBB within the brain, the blood-spinal cord barrier within the spinal cord, and the blood-retinal barrier within the retina are contiguous capillary barriers within the CNS, and are herein collectively referred to an the blood-brain barrier or BBB. The BBB also encompasses the blood-CSF barrier (choroid plexus) where the barrier is comprised of ependymal cells rather than capillary endothelial cells.

The knobs into holes dimerization modules and their use in antibody engineering are described in Carter P.; Ridgway J.B.B.; Presta L.G.: Immunotechnology, Volume 2, Number 1, February 1996 , pp. 73-73(1)).

The "central nervous system" or "CNS" refers to the complex of nerve tissues that control bodily function, and includes the brain and spinal cord.

A "blood-brain barrier receptor" (abbreviated "R/BBB" herein) is an extracellular membrane-linked receptor protein expressed on brain endothelial cells which is capable of transporting molecules across the BBB or be used to transport exogenous administrated molecules. Examples of R/BBB herein include: transferrin receptor (TfR), insulin receptor, insulin-like growth factor receptor (IGF-R), low density lipoprotein receptors including without limitation low density lipoprotein receptor-related protein 1 (LRP1) and low density lipoprotein receptor-related protein 8 (LRP8), and heparin-binding epidermal growth factor-like growth factor (HB-EGF). An exemplary R/BBB herein is transferrin receptor (TfR).

The "brain effector entity" refers to a molecule that is to be transported to the brain across the BBB. The effector entity typically has a characteristic therapeutic activity that is desired to be delivered to the brain. Effector entities include neurologically disorder drugs and cytotoxic agents such as e.g. peptides, proteins and antibodies, in particular monoclonal antibodies or fragments thereof directed to a brain target.

The "monovalent binding entity" refers to a molecule able to bind specifically and in a monovalent binding mode to an R/BBB. The blood brain shuttle of the present invention is characterized by the presence of a single unit of a monovalent binding entity i.e. the blood brain shuttle of the present invention comprises one unit of the monovalent binding entity. The monovalent binding entity includes but is not limited to proteins, polypeptides, peptides and antibody fragments including Fab, Fab', Fv fragments, single-chain antibody molecules such as e.g. single chain Fab, scFv. The monovalent binding entity can for example be a scaffold protein engineered using state of the art technologies like phage display or immunization. The monovalent binding entity can also be a peptide. The monovalent binding entity can comprises a CH2-CH3 Ig domain and a single Fab (sFab) directed to a blood brain barrier receptor. The scFab is coupled to the C-terminal end of the CH2-CH3 Ig domain by a linker. In certain embodiments, the sFab is directed to the transferrin receptor. The monovalent binding entity of the blood brain barrier shuttle of the invention is limited to "one scFab directed to the transferrin receptor".

The "monovalent binding mode" refers to a specific binding to the R/BBB where the interaction between the monovalent binding entity and the R/BBB take place through one single epitope. The monovalent binding mode prevents any dimerization/multimerization of the R/BBB due to a single epitope interaction point. The monovalent binding mode prevents that the intracellular sorting of the R/BBB is changed.

The term "epitope" includes any polypeptide determinant capable of specific binding to an antibody. The epitope determinant can include chemically active surface groupings of molecules such as amino acids, sugar side chains, phosphoryl, or sulfonyl, and may have specific three dimensional structural characteristics, and or specific charge characteristics. An epitope is a region of an antigen that is bound by an antibody.

The "transferrin receptor" ("TfR") is a transmembrane glycoprotein (with a molecular weight of about 180,000) composed of two disulphide-bonded sub-units (each of apparent molecular weight of about 90,000) involved in iron uptake in vertebrates. In one embodiment, the TfR herein is human TfR comprising the amino acid sequence as in Schneider et al. Nature 311 : 675 - 678 (1984), for example.

A "neurological disorder" as used herein refers to a disease or disorder which affects the CNS and/or which has an etiology in the CNS. Exemplary CNS diseases or disorders include, but are not limited to, neuropathy, amyloidosis, cancer, an ocular disease or disorder, viral or microbial infection, inflammation, ischemia, neurodegenerative disease, seizure, behavioral disorders, and a lysosomal storage disease. For the purposes of this specification, the CNS will be understood to include the eye, which is normally sequestered from the rest of the body by the blood-retina barrier. Specific examples of neurological disorders include, but are not limited to, neurodegenerative diseases (including, but not limited to, Lewy body disease, postpoliomyelitis syndrome, Shy-Draeger syndrome, olivopontocerebellar atrophy, Parkinson's disease, multiple system atrophy, striatonigral degeneration, tauopathies (including, but not limited to, Alzheimer disease and supranuclear palsy), prion diseases (including, but not limited to, bovine spongiform encephalopathy, scrapie, Creutzfeldt- Jakob syndrome, kuru, Gerstmann-Straussler-Scheinker disease, chronic wasting disease, and fatal familial insomnia), bulbar palsy, motor neuron disease, and nervous system heterodegenerative disorders (including, but not limited to, Canavan disease, Huntington's disease, neuronal ceroid- lipofuscinosis, Alexander's disease, Tourette's syndrome, Menkes kinky hair syndrome, Cockayne syndrome, Halervorden-Spatz syndrome, lafora disease, Rett syndrome, hepatolenticular degeneration, Lesch-Nyhan syndrome, and Unverricht-Lundborg syndrome), dementia (including, but not limited to, Pick's disease, and spinocerebellar ataxia), cancer (e.g. of the CNS and/or brain, including brain metastases resulting from cancer elsewhere in the body).

A "neurological disorder drug" is a drug or therapeutic agent that treats one or more neurological disorder(s). Neurological disorder drugs include, but are not limited to, small molecule compounds, antibodies, peptides, proteins, natural ligands of one or more CNS target(s), modified versions of natural ligands of one or more CNS target(s), aptamers, inhibitory nucleic acids (i.e., small inhibitory RNAs (siRNA) and short hairpin RNAs (shRNA)), ribozymes, and small molecules, or active fragments of any of the foregoing. Exemplary neurological disorder drugs are described herein and include, but are not limited to: antibodies, aptamers, proteins, peptides, inhibitory nucleic acids and small molecules and active fragments of any of the foregoing that either are themselves or specifically recognize and/or act upon (i.e., inhibit, activate, or detect) a CNS antigen or target molecule such as, but not limited to, amyloid precursor protein or portions thereof, amyloid beta, beta-secretase, gamma-secretase, tau, alpha-synuclein, parkin, huntingtin, DR6, presenilin, ApoE, glioma or other CNS cancer markers, and neurotrophins. Non-limiting examples of neurological disorder drugs and the corresponding disorders they may be used to treat: Brain-derived neurotrophic factor (BDNF), Chronic brain injury (Neurogenesis), Fibroblast growth factor 2 (FGF-2), Anti-Epidermal Growth Factor Receptor Brain cancer, (EGFR)-antibody, Glial cell-line derived neural factor Parkinson's disease, (GDNF), Brain-derived neurotrophic factor (BDNF) Amyotrophic lateral sclerosis, depression, Lysosomal enzyme Lysosomal storage disorders of the brain, Ciliary neurotrophic factor (CNTF) Amyotrophic lateral sclerosis, Neuregulin-1 Schizophrenia, Anti-HER2 antibody (e.g. trastuzumab) Brain metastasis from HER2 -positive cancer.

An "imaging agent" is a compound that has one or more properties that permit its presence and/or location to be detected directly or indirectly. Examples of such imaging agents include proteins and small molecule compounds incorporating a labeled entity that permits detection.

A "CNS antigen" or "brain target" is an antigen and/or molecule expressed in the CNS, including the brain, which can be targeted with an antibody or small molecule. Examples of such antigen and/or molecule include, without limitation: beta-secretase 1 (BACEI), amyloid beta (Abeta), epidermal growth factor receptor (EGFR), human epidermal growth factor receptor 2 (HER2), Tau, apolipoprotein E4 (ApoE4), alpha-synuclein, CD20, huntingtin, prion protein (PrP), leucine rich repeat kinase 2 (LRRK2), parkin, presenilin 1, presenilin 2, gamma secretase, death receptor 6 (DR6), amyloid precursor protein (APP), p75 neurotrophin receptor (p75NTR), and caspase 6. In one embodiment, the antigen is BACEI.

A "native sequence" protein herein refers to a protein comprising the amino acid sequence of a protein found in nature, including naturally occurring variants of the protein. The term as used herein includes the protein as isolated from a natural source thereof or as recombinantly produced.

The term "antibody" herein is used in the broadest sense and specifically covers monoclonal antibodies, polyclonal antibodies, multispecific antibodies {e.g. bispecific antibodies) formed from at least two intact antibodies, and antibody fragments so long as they exhibit the desired biological activity.

"Antibody fragments" herein comprise a portion of an intact antibody which retains the ability to bind antigen. Examples of antibody fragments include Fab, Fab', $F(ab')_2$, and Fv fragments; diabodies; linear antibodies; single-chain antibody molecules such as e.g. single chain Fab, scFv and multispecific antibodies formed from antibody fragments. The "Single chain Fab" format is e.g. described in Hust M. et al. BMC Biotechnol. 2007 Mar 8;7:14.

The term "monoclonal antibody" as used herein refers to an antibody obtained from a population of substantially homogeneous antibodies, i.e., the individual antibodies comprising the population are identical and/or bind the same epitope, except for possible variants that may arise during production of the monoclonal antibody, such variants generally being present in minor amounts. In contrast to polyclonal antibody preparations that typically include different antibodies directed against different determinants (epitopes), each monoclonal antibody is directed against a single determinant on the antigen. In addition to their specificity, the monoclonal antibodies are advantageous in that they are uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature, 256:495 (1975), or may be made by recombinant DNA methods (see, e.g., U.S. Patent No. 4,816,567). The "monoclonal antibodies" may also be isolated from phage antibody libraries using the techniques described in Clackson et al., Nature, 352:624-628 (1991) and Marks et al., J. Mol. Biol., 222:581-597 (1991), for example. Specific examples of monoclonal antibodies herein include chimeric antibodies, humanized antibodies, and human antibodies, including antigen-binding fragments thereof. The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al, Proc. Natl. Acad. Sci. USA, 81 :6851-6855 (1984)). Chimeric antibodies of interest herein include "primatized" antibodies comprising variable domain antigen-binding sequences derived from a non-human primate {e.g. Old World Monkey, such as baboon, rhesus or cynomolgus monkey) and human constant region sequences (US Pat No. 5,693,780).

"Humanized" forms of non-human {e.g., murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable regions correspond to those of a non-human immunoglobulin and all or substantially all of the FRs are those of a human immunoglobulin sequence, except for FR substitution(s) as noted above. The humanized antibody optionally also will comprise at least a portion of an immunoglobulin constant region, typically that of a human immunoglobulin. For further details, see Jones et al, Nature 321 :522-525 (1986); Riechmann et al, Nature 332:323-329 (1988); and Presta, Curr. Op. Struct. Biol 2:593-596 (1992).

A "human antibody" herein is one comprising an amino acid sequence structure that corresponds with the amino acid sequence structure of an antibody obtainable from a human B- cell, and includes antigen-binding fragments of human antibodies. Such antibodies can be identified or made by a variety of techniques, including, but not limited to: production by transgenic animals {e.g., mice) that are capable, upon immunization, of producing human antibodies in the absence of endogenous immunoglobulin production (see, e.g., Jakobovits et al, Proc. Natl Acad. Sci. USA, 90:2551 (1993); Jakobovits et al, Nature, 362:255-258 (1993); Bruggermann et al, Year in Immuno., 7:33 (1993); and US Patent Nos. 5,591,669, 5,589,369 and 5,545,807)); selection from phage display libraries expressing human antibodies or human antibody fragments (see, for example, McCafferty et al, Nature 348:552-553 (1990); Johnson et al, Current Opinion in Structural Biology 3:564-571 (1993); Clackson et al, Nature, 352:624-628 (1991); Marks et al, J. Mol. Biol. 222:581-597 (1991); Griffith et al, EMBO J. 12:725-734 (1993);US Patent Nos. 5,565,332 and 5,573,905); generation via in vitro activated B cells (see US Patents 5,567,610 and 5,229,275); and isolation from human antibody producing hybridomas.

A "multispecific antibody" herein is an antibody having binding specificities for at least two different epitopes. Exemplary multispecific antibodies may bind both an R/BBB and a brain antigen. Multispecific antibodies can be prepared as full-length antibodies or antibody fragments (e.g. F(ab')2 bispecific antibodies). Engineered antibodies with two, three or more (e.g. four) functional antigen binding sites are also contemplated (see, e.g., US Appln No. US 2002/0004587 Al, Miller et al.). Multispecific antibodies can be prepared as full length antibodies or antibody fragments. Several bispecific antibody formats, including the IgG-scFab format, are reviewed by Kontermann in "Dual targeting strategies with bispecific antibodies", MABS, vol. 4, no. 2, March 2012 (2012-03), pages 182-197.

Antibodies herein include "amino acid sequence variants" with altered antigen-binding or biological activity. Examples of such amino acid alterations include antibodies with enhanced affinity for antigen (e.g. "affinity matured" antibodies), and antibodies with altered Fc region, if present, e.g. with altered (increased or diminished) antibody dependent cellular cytotoxicity (ADCC) and/or complement dependent cytotoxicity (CDC) (see, for example, WO 00/42072, Presta, L. and WO 99/51642, Iduosogie et al); and/or increased or diminished serum half-life (see, for example, WO00/42072, Presta, L.).

An "affinity modified variant" has one or more substituted hypervariable region or framework residues of a parent antibody (e.g. of a parent chimeric, humanized, or human antibody) that alter (increase or reduce) affinity. The resulting variant(s) selected for further development will have reduced affinity for the R/BBB. A convenient way for generating such substitutional variants uses phage display. Briefly, several hypervariable region sites (e.g. 6-7 sites) are mutated to generate all possible amino substitutions at each site. The antibody variants thus generated are displayed in a monovalent fashion from filamentous phage particles as fusions to the gene III product of Ml 3 packaged within each particle. The phage-displayed variants are then screened for their biological activity (e.g. binding affinity). In order to identify candidate hypervariable region sites for modification, alanine scanning mutagenesis can be performed to identify hypervariable region residues contributing significantly to antigen binding. Alternatively, or additionally, it may be beneficial to analyze a crystal structure of the antigen-antibody complex to identify contact points between the antibody and its target. Such contact residues and neighboring residues are candidates for substitution according to the techniques elaborated herein. Once such variants are generated, the panel of variants is subjected to screening and antibodies with altered affinity may be selected for further development.

The antibody herein may be a "glycosylation variant" such that any carbohydrate attached to the Fc region, if present, is altered. For example, antibodies with a mature carbohydrate structure that lacks fucose attached to an Fc region of the antibody are described in US Pat Appl No US 2003/0157108 (Presta, L.). See also US 2004/0093621 (Kyowa Hakko Kogyo Co., Ltd). Antibodies with a bisecting N-acetylglucosamine (GlcNAc) in the carbohydrate attached to an Fc region of the antibody are referenced in WO 2003/011878, Jean-Mairet et al. and US Patent No. 6,602,684, Umana et al. Antibodies with at least one galactose residue in the oligosaccharide attached to an Fc region of the antibody are reported in WO 1997/30087, Patel et al. See, also, WO 1998/58964 (Raju, S.) and WO 1999/22764 (Raju, S.) concerning antibodies with altered carbohydrate attached to the Fc region thereof. See also US 2005/0123546 (Umana et al.) describing antibodies with modified glycosylation. The term "hypervariable region" when used herein refers to the amino acid residues of an antibody that are responsible for antigen binding. The hypervariable region comprises amino acid residues from a "complementarity determining region" or "CDR" (e.g. residues 24- 34 (LI), 50-56 (L2) and 89-97 (L3) in the light chain variable domain and 31-35 (HI), 50-65 (H2) and 95-102 (H3) in the heavy chain variable domain; Kabat et al., Sequences of Proteins of Immunological Interest, 5th Ed. Public Health Service, National Institutes of Health, Bethesda, MD. (1991)) and/or those residues from a "hypervariable loop" (e.g. residues 26-32 (LI), 50-52 (L2) and 91-96 (L3) in the light chain variable domain and 26-32 (HI), 53-55 (H2) and 96-101 (H3) in the heavy chain variable domain; Chothia and Lesk J. Mol. Biol. 196:901- 917 (1987)). "Framework" or "FR" residues are those variable domain residues other than the hypervariable region residues as herein defined.

A "full length antibody" is one which comprises an antigen-binding variable region as well as a light chain constant domain (CL) and heavy chain constant domains, CHI, CH2 and CH3. The constant domains may be native sequence constant domains (e.g. human native sequence constant domains) or amino acid sequence variants thereof.

A "naked antibody" is an antibody (as herein defined) that is not conjugated to a heterologous molecule, such as a cytotoxic entity, polymer, or radiolabel.

Antibody "effector functions" refer to those biological activities attributable to the Fc region (a native sequence Fc region or amino acid sequence variant Fc region) of an antibody. Examples of antibody effector functions include Clq binding, complement dependent cytotoxicity (CDC), Fc receptor binding, antibody-dependent cell-mediated cytotoxicity (ADCC), etc. The antibody may essentially lack effector function.

The term "antibody-dependent cellular cytotoxicity (ADCC)" refers to lysis of human target cells by an antibody in the presence of effector cells. The term "complement-dependent cytotoxicity (CDC)" denotes a process initiated by binding of complement factor C1q to the Fc part of most IgG antibody subclasses. Binding of C1q to an antibody is caused by defined protein-protein interactions at the so called binding site. Such Fc part binding sites are known in the state of the art. Such Fc part binding sites are, e.g., characterized by the amino acids L234, L235, D270, N297, E318, K320, K322, P331, and P329 (numbering according to EU index of Kabat). Antibodies of subclass IgG1, IgG2, and IgG3 usually show complement activation including C1q and C3 binding, whereas IgG4 does not activate the complement system and does not bind C1q and/or C3.

Depending on the amino acid sequence of the constant domain of their heavy chains, full length antibodies can be assigned to different "classes". There are five major classes of full length antibodies: IgA, IgD, IgE, IgG, and IgM, and several of these may be further divided into "subclasses" (isotypes), e.g., IgGl, IgG2, IgG3, IgG4, IgA, and IgA2. The heavy-chain constant domains that correspond to the different classes of antibodies are called alpha, delta, epsilon, gamma, and mu, respectively. The subunit structures and three-dimensional configurations of different classes of immunoglobulins are well known. The term "recombinant antibody", as used herein, refers to an antibody (e.g. a chimeric, humanized, or human antibody or antigen-binding fragment thereof) that is expressed by a recombinant host cell comprising nucleic acid encoding the antibody. Examples of "host cells" for producing recombinant antibodies include: (1) mammalian cells, for example, Chinese Hamster Ovary (CHO), COS, myeloma cells (including Y0 and NSO cells), baby hamster kidney (BHK), Hela and Vero cells; (2) insect cells, for example, sf9, sf21 and Tn5; (3) plant cells, for example plants belonging to the genus Nicotiana (e.g. Nicotiana tabacum); (4) yeast cells, for example, those belonging to the genus Saccharomyces (e.g. Saccharomyces cerevisiae) or the genus Aspergillus (e.g. Aspergillus niger); (5) bacterial cells, for example Escherichia, coli cells or Bacillus subtilis cells, etc.

As used herein, "specifically binding" or "binds specifically to" refers to an antibody selectively or preferentially binding to an antigen. The binding affinity is generally determined using a standard assay, such as Scatchard analysis, or surface plasmon resonance technique (e.g. using BIACORE®).

An "antibody that binds to the same epitope" as a reference antibody refers to an antibody that blocks binding of the reference antibody to its antigen in a competition assay by 50% or more, and conversely, the reference antibody blocks binding of the antibody to its antigen in a competition assay by 50% or more.

The term "cytotoxic agent" as used herein refers to a substance that inhibits or prevents a cellular function and/or causes cell death or destruction. Cytotoxic agents include, but are not limited to, radioactive isotopes (e.g., At211, 1131, 1125, Y90, Re186, Re188, Sm153, Bi212, P32, Pb212 and radioactive isotopes of Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof.

An "effective amount" of an agent, e.g., a pharmaceutical formulation, refers to an amount effective, at dosages and for periods of time necessary, to achieve the desired therapeutic or prophylactic result.

The term "Fc region" herein is used to define a C-terminal region of an immunoglobulin heavy chain that contains at least a portion of the constant region. The Fc region comprises the CH2 and CH3 domains of an immunoglobulin. The term includes native sequence Fc regions and variant Fc regions. The human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991. "Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The term "CH2-CH3 Ig entity" as used herein refers to a protein entity derived from immunoglobulin CH2 or CH3 domains. The "CH2-CH3 Ig entity" comprises two "CH2-CH3" polypeptides forming a dimer. The immunoglobulin can be IgG, IgA, IgD, IgE or IgM. The CH2-CH3 Ig entity may derive from an IgG immunoglobulin and is referred to herein as "CH2-CH3 IgG entity". The term includes native sequence of CH2-CH3 domains and variant CH2-CH3 domains. The "CH2-CH3 Ig entity" may derive from human heavy chain CH2-CH3 IgG domain which extends from Cys226, or from Pro230, to the carboxyl-terminus of the heavy chain. However, the C-terminal lysine (Lys447) of the Fc region may or may not be present. Unless otherwise specified herein, numbering of amino acid residues in the CH2-CH3 domain region or constant region is according to the EU numbering system, also called the EU index, as described in Kabat et al., Sequences of Proteins of Immunological Interest, 5th Ed. Public Health Service, National Institutes of Health, Bethesda, MD, 1991.

A "conjugate" is a fusion protein conjugated to one or more heterologous molecule(s), including but not limited to a label, neurological disorder drug or cytotoxic agent.

A "linker" as used herein refers to a chemical linker or a single chain peptide linker that covalently connects the different entities of the blood brain barrier shuttle and/or the fusion protein and/or the conjugate. The linker connects for example the brain effector entity to the monovalent binding entity. For example, if the monovalent binding entity comprises a CH2-CH3 Ig entity and a sFab directed to the blood brain barrier receptor, then the linker connects the sFab to the C-terminal end of the CH3-CH2 Ig entity. The linker connecting the brain effector entity to the monovalent binding entity (first linker) and the linker connecting the sFab to the C-terminal end of the CH2-CH3 Ig domain (second linker) can be the same or different.

Single chain peptide linkers, comprised of from one to twenty amino acids joined by peptide bonds, can be used. In certain embodiments, the amino acids are selected from the twenty naturally-occurring amino acids. In certain other embodiments, one or more of the amino acids are selected from glycine, alanine, proline, asparagine, glutamine and lysine. In other embodiments, the linker is a chemical linker. In certain embodiments, said linker is a single chain peptide with an amino acid sequence with a length of at least 25 amino acids, preferably with a length of 32 to 50 amino acids. In one embodiment said linker is (GxS)n with G = glycine, S = serine, (x =3, n= 8, 9 or 10 and m= 0, 1, 2 or 3) or (x = 4 and n= 6, 7 or 8 and m= 0, 1, 2 or 3), preferably with x = 4, n= 6 or 7 and m= 0, 1, 2 or 3, more preferably with x = 4, n= 7 and m= 2. In one embodiment said linker is $(G_4S)_4$ (Seq. Id. No. 17). In one embodiment said linker is $(G_4S)_6G_2$ (Seq. Id. No. 13).

Conjugation may be performed using a variety of chemical linkers. For example, the monovalent binding entity or the fusion protein and the brain effector entity may be conjugated using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HC1), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)- ethylenediamine), diisocyanates (such as toluene 2,6-diisocyanate), and bis-active fluorine compounds (such as I,5-difluoro-2,4-dinitrobenzene). The linker may be a "cleavable linker" facilitating release of the effector entity upon delivery to the brain. For example, an acid- labile linker, peptidase-sensitive linker, photolabile linker, dimethyl linker or disulfide- containing linker (Chari et al, Cancer Res. 52: 127-131 (1992); U.S. Patent No. 5,208,020) may be used.

Covalent conjugation can be either direct or via a linker. In certain embodiments, direct conjugation is by construction of a protein fusion (i.e., by genetic fusion of the two genes encoding the monovalent binding entity towards the R/BBB and effector entity and expressed as a single protein). In certain embodiments, direct conjugation is by formation of a covalent bond between a reactive group on one of the two portions of the monovalent binding entity against the R/BBB and a corresponding group or acceptor on the brain effector entity. In certain embodiments, direct conjugation is by modification (i.e., genetic modification) of one of the two molecules to be conjugated to include a reactive group (as non-limiting examples, a sulfhydryl group or a carboxyl group) that forms a covalent attachment to the other molecule to be conjugated under appropriate conditions. As one non-limiting example, a molecule (i.e., an amino acid) with a desired reactive group (i.e., a cysteine residue) may be introduced into, e.g., the monovalent binding entity towards the R/BBB antibody and a disulfide bond formed with the neurological drug. Methods for covalent conjugation of nucleic acids to proteins are also known in the art (i.e., photocrosslinking, see, e.g., Zatsepin et al. Russ. Chem. Rev. 74: 77-95 (2005)) Conjugation may also be performed using a variety of linkers. For example, a monovalent binding entity and a effector entity may be conjugated using a variety of bifunctional protein coupling agents such as N- succinimidyl-3-(2-pyridyldithio) propionate (SPDP), succinimidyl-4-(N-maleimidomethyl) cyclohexane-l-carboxylate (SMCC), iminothiolane (IT), bifunctional derivatives of imidoesters (such as dimethyl adipimidate HC1), active esters (such as disuccinimidyl suberate), aldehydes (such as glutaraldehyde), bis-azido compounds (such as bis (p- azidobenzoyl) hexanediamine), bis-diazonium derivatives (such as bis-(p-diazoniumbenzoyl)- ethylenediamine), diisocyanates (such as toluene 2,6-diisocyanate), and bis-active fluorine compounds (such as l,5-difluoro-2,4-dinitrobenzene). Peptide linkers, comprised of from one to twenty amino acids joined by peptide bonds, may also be used. In certain such embodiments, the amino acids are selected from the twenty naturally-occurring amino acids. In certain other such embodiments, one or more of the amino acids are selected from glycine, alanine, proline, asparagine, glutamine and lysine. The linker may be a "cleavable linker" facilitating release of the effector entity upon delivery to the brain. For example, an acid- labile linker, peptidase-sensitive linker, photolabile linker, dimethyl linker or disulfide- containing linker (Chari et al, Cancer Res. 52: 127-131 (1992); U.S. Patent No. 5,208,020) may be used.

A "label" is a marker coupled with the fusion protein herein and used for detection or imaging. Examples of such labels include: radiolabel, a fluorophore, a chromophore, or an affinity tag. The label may be a radiolabel used for medical imaging, for example tc99m or 1123, or a spin label for nuclear magnetic resonance (NMR) imaging (also known as magnetic resonance imaging, mri), such as iodine-123 again, iodine-131, indium- 111, fluorine- 19, carbon- 13, nitrogen-15, oxygen- 17, gadolinium, manganese, iron, etc. An "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the individual or subject is a human.

An "isolated" antibody is one which has been separated from a component of its natural environment. The antibody may be purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman et al, J. Chromatogr. B 848:79-87 (2007).

The term "package insert" is used to refer to instructions customarily included in commercial packages of therapeutic products, that contain information about the indications, usage, dosage, administration, combination therapy, contraindications and/or warnings concerning the use of such therapeutic products.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject., A pharmaceutically acceptable carrier includes, but is not limited to, a buffer, excipient, stabilizer, or preservative.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. The antibodies can be used to delay development of a disease or to slow the progression of a disease.

COMPOSITIONS AND METHODS

The methods and articles of manufacture disclosed herein, use, or incorporate, a blood brain barrier shuttle and/or fusion protein that binds to an R/BBB. The R/BBB antigen to be used for production of, or screening for, monovalent binding entity may be, e.g., a soluble form of or a portion thereof (e.g. the extracellular domain), containing the desired epitope. Alternatively, or additionally, cells expressing BBB- R at their cell surface can be used to generate, or screen for, monovalent binding entity. Other forms of R/BBB useful for generating monovalent binding entity will be apparent to those skilled in the art. Examples of R/BBB herein include transferrin receptor (TfR), insulin receptor, insulin-like growth factor receptor (IGF-R), low density lipoprotein receptor-related protein 1 (LRP1) and LRP8 and heparin-binding epidermal growth factor-like growth factor (HB-EGF).

According to the present disclosure, a "monovalent binding" entity against an R/BBB (e.g. monovalent binding entity for TfR) is selected based on the data herein demonstrating that such monovalent binding entity display improved CNS (for example, brain) uptake. In order to identify such binding entity, various assays for measuring monovalent binding mode are available including, without limitation: Scatchard assay and surface plasmon resonance technique (e.g. using BIACORE®) and in vivo investigations described herein.

The disclosure provides a method of making a monovalent binding entity useful for transporting an brain effector entity such as e.g. a neurological disorder drug, across the blood-brain barrier comprising selecting a monovalent binding entity from a panel of monovalent binding moieties against an R/BBB because it has an monovalent binding mode for the R/BBB. The monovalent binding mode ensures efficient BBB crossing for certain R/BBB by not interfering with the receptors normal intracellular sorting.

Another group of compounds that may be selected as neurological drugs for cancer treatment or prevention are anti-cancer immunoglobulins (including, but not limited to, trastuzumab, bevacizumab, alemtuxumab, cetuximab, gemtuzumab ozogamicin, ibritumomab tiuxetan, panitumumab and rituximab). In some instances, antibodies in conjunction with a toxic label may be used to target and kill desired cells (i.e., cancer cells), including, but not limited to, tositumomab with a radiolabel.

The brain effector entity may be an intact or full-length antibody. Depending on the amino acid sequence of the constant domain of their heavy chains, intact antibodies can be assigned to different classes. There are five major classes of intact antibodies: IgA, IgD, IgE, IgG, and IgM, and several of these may be further divided into subclasses (isotypes), e.g., IgGl, IgG2, IgG3, IgG4, IgA, and IgA2. The heavy chain constant domains that correspond to the different classes of antibodies are called $\alpha, \delta, \varepsilon, \gamma$, and $\mu$, respectively. The subunit structures and three-dimensional configurations of different classes of immunoglobulins are well known. The intact antibody can lack effector function.

Techniques for generating antibodies are known and examples provided above in the definitions section of this document. The antibody can be a chimeric, humanized, or human antibody or antigen-binding fragment thereof.

Various techniques are available for determining binding of the monovalent binding entity to the R/BBB. One such assay is an enzyme linked immunosorbent assay (ELIS A) for confirming an ability to bind to human R/BBB (and brain antigen). According to this assay, plates coated with antigen (e.g. recombinant sR/BBB) are incubated with a sample comprising the monovalent binding entity towards the R/BBB and binding of the monovalent binding entity to the antigen of interest is determined.

In one aspect, the monovalent binding entity of the disclosure is tested for its antigen binding activity, e.g., by known methods such as ELISA, Western blot, etc.

In one aspect, the monovalent binding entity of the disclosure is tested for its single antigen binding activity towards an R/BBB using epitope mapping of X-ray structure determination.

Assays for evaluating uptake of systemically administered blood brain barrier shuttle and/or conjugate and other biological activity of blood brain barrier shuttle and/or conjugate can be performed as disclosed in the examples or as known for the blood brain barrier shuttle and/or conjugate of interest. Measuring the concentration within the parenchyma space of CNS can also be used using for example microdialysis or the capillary depletion method combined with ELISA or radioactivity measurements of labeled blood brain barrier shuttle and/or conjugate.

PHARMACEUTICAL FORMULATIONS

Therapeutic formulations of the blood brain barrier shuttle and/or conjugate used in accordance with the present disclosure are prepared for storage by mixing with optional pharmaceutically acceptable carriers, excipients or stabilizers {Remington 's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN™, PLURONICS™ or polyethylene glycol (PEG).

The formulation herein may also contain more than one active compound as necessary, optionally those with complementary activities that do not adversely affect each other. The type and effective amounts of such medicaments depend, for example, on the amount of blood brain barrier shuttle and/or conjugate present in the formulation, and clinical parameters of the subjects. Exemplary such medicaments are discussed below.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained- release preparations include semi-permeable matrices of solid hydrophobic polymers containing the antibody, which matrices are in the form of shaped articles, e.g. films, or microcapsules. Examples of sustained-release matrices include polyesters, hydrogels (for example, poly(2-hydroxyethylmethacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid.

The formulations to be used for in vivo administration must be sterile. This is readily accomplished by filtration through sterile filtration membranes. In one embodiment the formulation is isotonic.

The blood brain barrier shuttle of the invention may be utilized in a variety of in vivo methods. For example, the disclosure provides a method of transporting a therapeutic compound across the BBB comprising exposing the blood brain barrier shuttle and/or conjugate to the BBB such that the monovalent binding entity transports the therapeutic compound coupled thereto across the BBB. In another example, the disclosure provides a method of transporting a neurological disorder drug across the BBB comprising exposing the blood brain barrier shuttle and/or conjugate to the BBB such that the monovalent binding entity transports the neurological disorder drug coupled thereto across the BBB. In another example, the BBB here is in a mammal (e.g. a human), e.g. one which has a neurological disorder, including, without limitation: Alzheimer's disease (AD), stroke, dementia, muscular dystrophy (MD), multiple sclerosis (MS), amyotrophic lateral sclerosis (ALS), cystic fibrosis, Angelman's syndrome, Liddle syndrome, Parkinson's disease, Pick's disease, Paget's disease, cancer, traumatic brain injury, etc.

A neurological disorder is selected from: a neuropathy, amyloidosis, cancer (e.g. involving the CNS or brain), an ocular disease or disorder, a viral or microbial infection, inflammation (e.g. of the CNS or brain), ischemia, neurodegenerative disease, seizure, behavioral disorder, lysosomal storage disease, etc.

Neuropathy disorders are diseases or abnormalities of the nervous system characterized by inappropriate or uncontrolled nerve signaling or lack thereof, and include, but are not limited to, chronic pain (including nociceptive pain), pain caused by an injury to body tissues, including cancer-related pain, neuropathic pain (pain caused by abnormalities in the nerves, spinal cord, or brain), and psychogenic pain (entirely or mostly related to a psychological disorder), headache, migraine, neuropathy, and symptoms and syndromes often accompanying such neuropathy disorders such as vertigo or nausea.

Amyloidoses are a group of diseases and disorders associated with extracellular proteinaceous deposits in the CNS, including, but not limited to, secondary amyloidosis, age- related amyloidosis, Alzheimer's Disease (AD), mild cognitive impairment (MCI), Lewy body dementia, Down's syndrome, hereditary cerebral hemorrhage with amyloidosis (Dutch type); the Guam Parkinson-Dementia complex, cerebral amyloid angiopathy, Huntington's disease, progressive supranuclear palsy, multiple sclerosis; Creutzfeld Jacob disease, Parkinson's disease, transmissible spongiform encephalopathy, HIV-related dementia, amyotropic lateral sclerosis (ALS), inclusion-body myositis (IBM), and ocular diseases relating to beta-amyloid deposition (i.e., macular degeneration, drusen-related optic neuropathy, and cataract).

Cancers of the CNS are characterized by aberrant proliferation of one or more CNS cell (i.e., a neural cell) and include, but are not limited to, glioma, glioblastoma multiforme, meningioma, astrocytoma, acoustic neuroma, chondroma, oligodendroglioma, meduUoblastomas, ganglioglioma, Schwannoma, neurofibroma, neuroblastoma, and extradural, intramedullary or intra-dural tumors.

Viral or microbial infections of the CNS include, but are not limited to, infections by viruses (i.e., influenza, HIV, poliovirus, rubella, ), bacteria (i.e., Neisseria sp., Streptococcus sp., Pseudomonas sp., Proteus sp., E. coli, S. aureus, Pneumococcus sp., Meningococcus sp., Haemophilus sp., and Mycobacterium tuberculosis) and other microorganisms such as fungi (i.e., yeast, Cryptococcus neoformans), parasites (i.e., toxoplasma gondii) or amoebas resulting in CNS pathophysiologies including, but not limited to, meningitis, encephalitis, myelitis, vasculitis and abscess, which can be acute or chronic. Inflammation of the CNS is inflammation that is caused by an injury to the CNS, which can be a physical injury (i.e., due to accident, surgery, brain trauma, spinal cord injury, concussion) or an injury due to or related to one or more other diseases or disorders of the CNS (i.e., abscess, cancer, viral or microbial infection).

Ischemia of the CNS, as used herein, refers to a group of disorders relating to aberrant blood flow or vascular behavior in the brain or the causes therefor, and includes, but is not limited to: focal brain ischemia, global brain ischemia, stroke (i.e., subarachnoid hemorrhage and intracerebral hemorrhage), and aneurysm.

Neurodegenerative diseases are a group of diseases and disorders associated with neural cell loss of function or death in the CNS, and include, but are not limited to: adrenoleukodystrophy, Alexander's disease, Alper's disease, amyotrophic lateral sclerosis, ataxia telangiectasia, Batten disease, cockayne syndrome, corticobasal degeneration, degeneration caused by or associated with an amyloidosis, Friedreich's ataxia, frontotemporal lobar degeneration, Kennedy's disease, multiple system atrophy, multiple sclerosis, primary lateral sclerosis, progressive supranuclear palsy, spinal muscular atrophy, transverse myelitis, Refsum's disease, and spinocerebellar ataxia.

Seizure diseases and disorders of the CNS involve inappropriate and/or abnormal electrical conduction in the CNS, and include, but are not limited to: epilepsy (i.e., absence seizures, atonic seizures, benign Rolandic epilepsy, childhood absence, clonic seizures, complex partial seizures, frontal lobe epilepsy, febrile seizures, infantile spasms, juvenile myoclonic epilepsy, juvenile absence epilepsy, Lennox-Gastaut syndrome, Landau-Kleffner Syndrome, Dravet's syndrome, Otahara syndrome, West syndrome, myoclonic seizures, mitochondrial disorders, progressive myoclonic epilepsies, psychogenic seizures, reflex epilepsy, Rasmussen's Syndrome, simple partial seizures, secondarily generalized seizures, temporal lobe epilepsy, toniclonic seizures, tonic seizures, psychomotor seizures, limbic epilepsy, partial-onset seizures, generalized-onset seizures, status epilepticus, abdominal epilepsy, akinetic seizures, autonomic seizures, massive bilateral myoclonus, catamenial epilepsy, drop seizures, emotional seizures, focal seizures, gelastic seizures, Jacksonian March, Lafora Disease, motor seizures, multifocal seizures, nocturnal seizures, photosensitive seizure, pseudo seizures, sensory seizures, subtle seizures, sylvan seizures, withdrawal seizures, and visual reflex seizures).

Behavioral disorders are disorders of the CNS characterized by aberrant behavior on the part of the afflicted subject and include, but are not limited to: sleep disorders (i.e., insomnia, parasomnias, night terrors, circadian rhythm sleep disorders, and narcolepsy), mood disorders (i.e., depression, suicidal depression, anxiety, chronic affective disorders, phobias, panic attacks, obsessive-compulsive disorder, attention deficit hyperactivity disorder (ADHD), attention deficit disorder (ADD), chronic fatigue syndrome, agoraphobia, post-traumatic stress disorder, bipolar disorder), eating disorders (i.e., anorexia or bulimia), psychoses, developmental behavioral disorders (i.e., autism, Rett's syndrome, Aspberger's syndrome), personality disorders and psychotic disorders (i.e., schizophrenia, delusional disorder, and the like).

Lysosomal storage disorders are metabolic disorders which are in some cases associated with the CNS or have CNS-specific symptoms; such disorders include, but are not limited to: Tay-Sachs disease, Gaucher's disease, Fabry disease, mucopolysaccharidosis (types I, II, III, IV, V, VI and VII), glycogen storage disease, GM1 -gangliosidosis, metachromatic leukodystrophy, Farber's disease, Canavan's leukodystrophy, and neuronal ceroid lipofuscinoses types 1 and 2, Niemann-Pick disease, Pompe disease, and Krabbe's disease.

The blood brain barrier shuttle of the invention can be used either alone or in combination with other agents in a therapy. For instance, the blood brain barrier shuttle of the invention may be co-administered with at least one additional therapeutic agent. An additional therapeutic agent is a therapeutic agent effective to treat the same or a different neurological disorder as the blood brain barrier shuttle of the invention is being employed to treat. Exemplary additional therapeutic agents include, but are not limited to: the various neurological drugs described above, cholinesterase inhibitors (such as donepezil, galantamine, rovastigmine, and tacrine), NMDA receptor antagonists (such as memantine), amyloid beta peptide aggregation inhibitors, antioxidants, γ-secretase modulators, nerve growth factor (NGF) mimics or NGF gene therapy, PPARy agonists, HMS-CoA reductase inhibitors (statins), ampakines, calcium channel blockers, GABA receptor antagonists, glycogen synthase kinase inhibitors, intravenous immunoglobulin, muscarinic receptor agonists, nicrotinic receptor modulators, active or passive amyloid beta peptide immunization, phosphodiesterase inhibitors, serotonin receptor antagonists and anti-amyloid beta peptide antibodies. In certain embodiments, the at least one additional therapeutic agent is selected for its ability to mitigate one or more side effects of the neurological drug.

Such combination therapies noted above encompass combined administration (where two or more therapeutic agents are included in the same or separate formulations), and separate administration, in which case, administration of the blood brain barrier shuttle of the invention can occur prior to, simultaneously, and/or following, administration of the additional therapeutic agent and/or adjuvant. Blood brain barrier shuttles of the invention can also be used in combination with other interventional therapies such as, but not limited to, radiation therapy, behavioral therapy, or other therapies known in the art and appropriate for the neurological disorder to be treated or prevented. The blood brain barrier shuttle of the invention (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration.

Dosing can be by any suitable route, e.g. by injections, such as intravenous or subcutaneous injections, depending in part on whether the administration is brief or chronic. Various dosing schedules including but not limited to monovalent or multiple administrations over various time- points, bolus administration, and pulse infusion are contemplated herein.

Blood brain barrier shuttle of the invention would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The blood brain barrier shuttle of the invention need not be, but is optionally formulated with one or more agents currently used to prevent or treat the disorder in question. The effective amount of such other agents depends on the amount of blood brain barrier shuttle and/or conjugate present in the formulation, the type of disorder or treatment, and other factors discussed above. These are generally used in the same dosages and with administration routes as described herein, or about from 1 to 99% of the dosages described herein, or in any dosage and by any route that is empirically/-clinically determined to be appropriate.

For the prevention or treatment of disease, the appropriate dosage of blood brain barrier shuttle of the invention (when used alone or in combination with one or more other additional therapeutic agents) will depend on the type of disease to be treated, the type of blood brain barrier shuttle and/or conjugate, the severity and course of the disease, whether the antibody is administered for preventive or therapeutic purposes, previous therapy, the patient's clinical history and response to the blood brain barrier shuttle and/or conjugate, and the discretion of the attending physician. The blood brain barrier shuttle and/or conjugate is suitably administered to the patient at one time or over a series of treatments. Depending on the type and severity of the disease, about 1 μg/kg to 15 mg/kg (e.g. 0.1 mg/kg- 10mg/kg) of blood brain barrier shuttle and/or conjugate can be an initial candidate dosage for administration to the patient, whether, for example, by one or more separate administrations, or by continuous inmultimeric. One typical daily dosage might range from about 1 μg/kg to 100 mg/kg or more, depending on the factors mentioned above. For repeated administrations over several days or longer, depending on the condition, the treatment would generally be sustained until a desired suppression of disease symptoms occurs. One exemplary dosage of the antibody would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (e.g. such that the patient receives from about two to about twenty, or e.g. about six doses of the antibody). An initial higher loading dose, followed by one or more lower doses may be administered. However, other dosage regimens may be useful. The progress of this therapy is easily monitored by conventional techniques and assays.

EXAMPLES

## Example 1: Generation of the expression plasmids

Description of the basic/standard mammalian expression plasmid

Desired proteins were expressed by transient transfection of human embryonic kidney cells (HEK 293). For the expression of a desired gene/protein (e.g. antibody-Fab multimeric protein) a transcription unit comprising the following functional elements was used:

- the immediate early enhancer and promoter from the human cytomegalovirus (P-CMV) including intron A,

- a human heavy chain immunoglobulin 5'-untranslated region (5'UTR),

- a murine immunoglobulin heavy chain signal sequence (SS),

- a gene/protein to be expressed (e.g. full length antibody heavy chain), and

- the bovine growth hormone polyadenylation sequence (BGH pA).

Beside the expression unit/cassette including the desired gene to be expressed the basic/standard mammalian expression plasmid contains:

- an origin of replication from the vector pUC18 which allows replication of this plasmid in E. coli, and

- a beta-lactamase gene which confers ampicillin resistance in E. coli.

## Expression plasmids coding for the following antibody-sFab fusion polypeptides/proteins were constructed:

*Tetravalent Mab31-scFab(8D3) (Fig. 1C) (Mab31 = human monoclonal antibody recognizing Abeta. INN of Mab 31 = Gantenerumab)*

Heavy chain (10132_pPM284_Mab31(IgG1)-$(G_4S)_4$-VL-Ck-$(G_4S)_6$-GG-VH-CH1) (Seq. Id. No. 1)

Composition of the Mab31-scFab(8D3) heavy chain fusion protein:

- Mab31 human IgG1 heavy chain without C-terminal Lys

- Glycine Serine-linker

- Variable light chain domain (VL) variant (L596V and L598I) of the mouse 8D3 anti-transferrin antibody (Boado, R.J. Zhang, Y. Wang, Y and Pardridge, W.M., Biotechnology and Bioengineering (2009) 102, 1251-1258)

- Human C-kappa light chain

- GlycineSerine-linker

- Variable heavy chain domain (VH) of the mouse 8D3 anti-transferrin antibody (Boado, R.J. Zhang, Y. Wang, Y and Pardridge, W.M., Biotechnology and Bioengineering (2009) 102, 1251-1258)

- Human IgG1 CH3 heavy chain domain

Light chain (5170-VL-Mab31-BsmI-L2-Neo-BGHpA) (Seq. Id. No. 2)

Composition of the Mab31 light chain

- Mab31 human Ckappa light chain

*Trivalent Mab31-scFab(8D3) (Fig. 1B)*

Knob heavy chain (10134_pPM287_Mab31(IgG1)_knob_SS_-(G$_4$S)$_4$-VL-Ck-(G$_4$S)$_6$-GG-VH-CH1) (Seq. Id. No. 3)

Composition of the knob Mab31-scFab(8D3) heavy chain fusion protein

- Mab31 human IgG1 heavy chain without C-terminal Lys containing the CH3 knob mutation T366W and the S354C mutation for the formation of an additional disulfide bridge

- GlycineSerine-linker

- Variable light chain domain (VL) variant (L596V and L598I) of the mouse 8D3 anti-transferrin antibody (Boado, R.J. Zhang, Y. Wang, Y and Pardridge, W.M., Biotechnology and Bioengineering (2009) 102, 1251-1258)

- Human C-kappa light chain

- GlycineSerine-linker

- Variable heavy chain domain (VH) of the mouse 8D3 anti-transferrin antibody (Boado, R.J. Zhang, Y. Wang, Y and Pardridge, W.M., Biotechnology and Bioengineering (2009) 102, 1251-1258)

- Human IgG1 CH3 heavy chain domain

Hole heavy chain (10133_pPM286_Mab31(IgG1)_hole_SS) (Seq. Id. No. 4)

Composition of the hole Mab31 heavy chain fusion protein

- Mab31 human IgG1 heavy chain containing the CH3 hole mutations T366S, Y407V and L368A and the Y349C mutation for the formation of an additional disulfide bridge

Light chain (5170-VL-Mab31-BsmI-L2-Neo-BGHpA) (Seq. Id. No. 2)

Composition of the Mab31 light chain

- Mab31 human Ckappa light chain

## Example 2: Purification of single and double Mab31-Fab constructs

The antibody chains were generated by transient transfection of HEK293 cells (human embryonic kidney cell line 293-derived) cultivated in F17 Medium (Invitrogen Corp.). For transfection "293-Fectin" Transfection Reagent (Invitrogen) was used. The antibody chains were expressed from two (tetravalent Mab31-scFab(8D3)) or three (trivalent Mab31-scFab(8D3)) different plasmids, coding for the tetravalent Mab31-scFab(8D3) heavy chain and the Mab31 corresponding light chain, or the knob and hole trivalent Mab31-scFab(8D3) heavy chains and the Mab31 corresponding light chain, respectively. The two or three plasmids were used at an equimolar plasmid ratio upon transfection. Transfections were performed as specified in the manufacturer's instructions. Antibody fusion proteins-containing cell culture supernatants were harvested seven days after transfection. Supernatants were stored frozen until purification.

Proteins were purified from filtered cell culture supernatants. Supernatants were applied to a protein A Sepharose column (GE Healthcare) and washed with PBS pH 7.4. Elution of antibodies was achieved with 100 mM Citate buffer at pH 3.0 followed by immediate neutralization of the sample to pH6.5. After concentration aggregated protein and other byproducts were separated from monomeric antibodies by size exclusion chromatography (Superdex 200; GE Healthcare) in 20 mM histidine, 140 mM NaCl, pH 6.0. Every single fraction was analyzed on analytical SEC (TSK G3000SWXL) and on a chip-based capillary electrophoresis system (CE-SDS, LabChipGX, Caliper) for the quantification of incompletely assembled molecules and other byproducts. Monomeric antibody fractions without byproducts were pooled. After concentration using a MILLIPOREAmicon Ultra (30 molecular weight cut off) centrifugal concentrator the protein was stored at -80 °C. Analytical characterization of the endproduct was done by UV protein determination, CE-SDS, size-exclusion chromatography, mass spectrometry and also by endotoxin determination.

## Example 3: ELISA binding data of single Fab and double Fab constructs

Binding of mAb31-8D3 constructs to mouse transferrin receptor (mTfR) was assessed by indirect ELISA. To this end, recombinant mTfR (extracellular domain; Sino Biological) was coated to Maxisorb microtiter plate (Nunc) at 1 $\mu$g/mL in PBS at 4°C overnight. After blocking in 1% Crotein-C/PBS (blocking buffer; Roche) for 1 h at RT and 4 washes with 0.1% Tween-20/PBS (wash buffer), mAb31-8D3 constructs were added to the wells at concentrations between 0.01 and 150 nM in blocking buffer and incubated for 1 h at RT. After 4 wash steps, constructs were detected by addition of anti-human-IgG-HRP (Jackson Immunoresearch) at 1:10,000 dilution in blocking buffer (1 RT), followed by 6 washes and incubation in TMB (Sigma). Absorbance was read out at 450 nm after stopping color development with 1 N HCl.

Fig. 3 shows that binding of the bivalent mAb31-8D3-dFab to mTfR is comparable to that of 8D3 IgG, while the monovalent construct mAb31-8D3-sFab shows a reduced affinity.

Functionality of mAb31 was confirmed by ELISA. Briefly, Abeta(1-40) was coated at 7 $\mu$g/mL in PBS onto Maxisorp plates for 3 days at 37°C to produce fibrillar Abeta, then dried for 3 h at RT. The plate was blocked with 1% Crotein C and 0.1% RSA in PBS (blocking buffer) for 1 h at RT, then washed once with wash buffer. mAb31 constructs were added at concentrations up to 100 nM in blocking buffer and incubated at 4°C overnight. After 4 wash steps, constructs were detected as indicated above.

Fig. 4 shows that both mAb31-8D3 constructs (sFab and dFab) bind with an affinity comparable to that of unmodified mAb31 to immobilized Abeta fibrils.

## Example 4: Only single Fab constructs cross the BBB and decorates plaques

Brain sectioning and immunohistochemical staining:

Brains were prepared after PBS perfusion and sagittal cryo-sections were cut between lateral ~ 1.92 and 1.68 millimeter according to the brain atlas of Paxinos and Franklin. Brains were sectioned at a nominal thickness of 20 microns at -15°C using a Leica CM3050 S cryostat and placed onto precooled glass slides (Superfrost plus, Menzel, Germany). For each brain, three sections spaced 80 microns were deposited on the same slide.

Sections were rehydrated in PBS for 5 minutes followed by immersion with 100% acetone precooled to -20°C for 2 min. All further steps were done at room temperature. Slides with brain sections were washed with PBS, pH 7.4 and blocking of unspecific binding sites by sequential incubation in Ultra V block (LabVision) for 5 minutes followed by PBS wash and incubation in power block solution (BioGenex) with 2 % normal goat serum in PBS for 20 min. Slides were directly incubated with the secondary antibody, an affinity-purified goat anti-human IgG (heavy and light chain specific) conjugated to Alexa Fluor 555 dye (# A-21433, lot 54699A, Molecular Probes) at a concentration of 20 microg/ml in 2 % normal goat serum in PBS, pH 7.4 for 1 hour. After extensive washing with PBS, plaque localization was assessed by a double-labeling for Abeta plaques by incubation with BAP-2, a Roche in-house murine monoclonal antibody against Abeta conjugated to Alexa Fluor 488 dye at 0.5 microg /ml for 1 hour in PBS with power block solution (BioGenex) and 10 % normal sheep serum. After PBS washing, autofluorescence of lipofuscin was reduced by quenching through incubation in 4mM CuSO4 in 50 mM ammonium acetate, pH 5 for 30 minutes. After rinsing the slides with double-distilled water, slides were embedded with Confocal Matrix (Micro Tech Lab, Austria).

Confocal microscopy

Three images from each section of the brain of each PS2APP-mouse with plaque containing regions in the frontal cortex (region of the primary motor cortex) were taken. Images were recorded with a Leica TCS SP5 confocal system with a pinhole setting of 1 Airy.

Plaques immunolabelled with Alexa Fluor 488 dyes were captured in the same spectral conditions (a 488nm excitation and a 500-554nm band pass emission) with adjusted photomultiplier gain and offset (typically, 770 V and -0% respectively) at a 30% laser power.

Bound secondary Alexa Fluor 555 antibodies on the accessible surface of tissue sections were recorded at the 561 nm excitation laser line at a window ranging from 570 to 725 nm covering the emission wavelength range of the applied detection antibody. Instrument settings were kept constant for image acquisitions to allow comparative intensity measurements for tested human anti-A$\beta$ antibodies; in particular, laser power, scanning speed, gain and offset. Laser power was set to 30% and settings for PMT gain were typically 850 V and a nominal offset of 0%. This enabled visualization of both faint and strongly stained plaques with the same setting. Acquisition frequency was at 400 Hz.

Confocal scans were recorded as single optical layers with a HCX PL APO 20x 0.7 IMM UV objective in water, at a 512 x 512 pixel resolution and an optical measuring depth in the vertical axis was interactively controlled to ensure imaging within the tissue section. Amyloid-□ plaques located in layers 2-5 of the frontal cortex were imaged and fluorescent intensities quantified.

Statistical analysis

Immunopositive regions were visualized as TIFF images and processed for quantification of fluorescence intensity and area (measured in pixels) with ImageJ version 1.45 (NIH). For quantification, background intensities of 5 were subtracted in every image and positive regions smaller than 5 square pixels were filtered out. Total fluorescence intensity of selected isosurfaces was determined as sum of intensities of single individual positive regions and the mean pixel intensity was calculated dividing the total intensity by the number of pixels analyzed.

Average and standard deviations values were calculated with Microsoft Excel (Redmond / WA, USA) from all measured isosurfaces obtained from nine pictures taken from three different sections for each animal. Statistical analysis was performed using the Student's t test for group comparison or a Mann-Whitney test.

10 mg/ml of mAb31 (construct of Fig1A), 13.3 mg/kg sFab-mAb31 (construct of Fig1B) and 16.7 mg/kg of dFab-mAb31 (construct of Fig1C) was i.v. tail injected in mice and after 8 hours the brain was perfused with PBS. Sections was prepared as described above and stained with the goat anti-human IgG. For the mAb31 construct almost no specific signal was detected (Fig 4A). For the sFab-mAb31 expensive staining of both the plaque and capillaries was detected (Fig. 4B) while the dFab-mAb31 only staining of the capillaries was detected (Fig 4C). This cleary showed that a monovalent binding mode (sFab-mAb31 to the Transferrin receptor is much more efficient bring the construct through the brain endothelial cells at the BBB. The quantification of the bivalent binding molecule (dFab-mAb31) is shown in Fig 5. The data shows that there is not increase in plaque decoration for the dFab-mAb31 construct, there is only an increase in total intensity due to the capillary accumulation of the construct.

**Example 5: Quantification of brain exposures with a single Fab construct**

The experimental procedure is described in Example 4. Quantification of the sFab-mAb31 brain exposure is shown in Fig 6 using 10 mg/ml of mAb31 (construct of Fig1A) and 13.3 mg/kg sFab-mAb31 (construct of Fig1B). Already 8 hours after the injection of the sFab-mAb31 construct there is a massive uptake compare to mAb31 (about 55-fold increase). Similar data was obtained after 24 hours post dose using 25 mg/ml of mAb31 (construct of Fig1A) and 33.3 mg/kg sFab-mAb31 (construct of Fig1B). Fig 6 also shows the transient capillary staining of the sFab-mAb31 illustrates the targeting effect and the crossing of the BBB over time. All these data are highly significant as indicated in Fig 6.

Fig 7 shows data of the mAb31 (construct of Fig. 1A) and the sFab-mAb31 (construct of Fig. 1B) construct at a low dose. Again only the sFab-mAb31 construct is able to cross the brain endothelial cells and decorate the plaque in the brain. Maximal effect is already reached at 8 hours post dose. It is only at a higher dose (10 mg/kg) and relative long time (7 days) for the mAb31 construct that there is a trend for increase in the signal of binding to the Abeta plaques in the brain (Fig 7). All these data are highly significant as indicated in Fig 7.

**Example 6: Specific down-regulation of cell surface TfR by a double Fab construct**

Experimental details: bEnd3 cells cultured in a 6-well plate format. 2-3 days after confluence treated with dFab-mAb31, sFab-mAb31 or untreated ctr. for 24 hours. Then medium removed/aspirated and cells washed twice with ice cold PBS (-MgCl)(-CaCl) (Gibco 14190-094), 5ml/well. 1ml Trypsin/EDTA (Lonza CC-5012)/ well were added, incubated at 37°C for 15 minutes until all cells were detached. Stopped reaction with 1ml trypsin neutralizing solution (ice-cold) (Lonza CC-5002). 2ml of the Trypsin/EDTA + neutralization solution collected in a 50ml Falcon tube and kept on ice. Centrifugation of the cells at 4°C with 1400 rpm for 10 minutes. Pellets re-suspended in 50ml ice cold bEnd3 Medium (DMEM-12 (Gibco 31331) + 10%FBS). Centrifugation of the cells at 4°C with 1400rpm for 10 minutes. Pellet re-suspended in 3ml ice cold FACS-Buffer (BD 554656). Cell counts: a) sFab tube ($2,5x10^5$ cells/ml) viability: 47%, b) dFab tube ($3.18x10^5$ cells/ml) viability: 55%, c) ctr. tube ($4.6x10^5$ cells/ml) viability: 57%. FACS staining $1x10^5$ cells/eppendorf tube distributed and centrifuged (4°C,10min,1500rpm). Supernatant aspirated; a) CD71-PE (clone R17217- IgG2a monoclonal) (santa cruz sc-52504) 20 microL of the antibody/pellet (staining volume 100microL) filled up to 100 microL with ice cold FACS-Buffer (BD 554656), b) CD31-APC (BD 551262) (rat anti mouse IgG2a (200 microg/ml)) 5 microg antibody/pellet (staining volume 100 microL) filled up to 100 microL with ice cold FACS-Buffer (BD 554656), c) 8D3-Alexa488 (1:50) (staining volume 100 microL) diluted in ice cold FACS-Buffer (BD 554656), d) Isotype ctr. for Alexa488, APC and PE (all from BD). Incubation in the dark at ice for 1 hour. Filled up to 1.5 ml with ice cold FACS-Buffer and centrifuged (4°C,10 min,1500rpm). Washed pellet twice with 1.5 ml ice cold FACS-Buffer and finally re-suspended pellet in 500 microL PBS. FACS measurement was performed using the instrument Guava Flow Cytometry. The data shows that the double (dFab) construct (Fig 8B) appears to down-regulate the Transferrin receptor on the cell surface. This is not detectable in this assay setup with the single (sFab) construct (Fig 8A) indicating that a monovalent binding mode has no direct effect on the cell trafficking and recycling that determine the amount of the Transferrin receptor at the cell surface on brain endothelial cells.

**Example 7: In vivo intracellular sorting of a single and double Fab construct**

APPswe/PS2 transgenic mice were injected i.v (tail injection) with the following constructs MAb31 (10mg/kg), sFab-MAb31 (13.3 mg/kg) or dFab-MAb31 (17.44mg/kg). The injected dose reflects the molecule size with MAb31 used as reference. 15 minutes or 8 hours after the injection, mice were euthanized with $CO_2$ and treated as followed. The right cardiac atrium of the heart was cut open so that blood and perfusion solution can flow out. The left cardiac ventricle was incised and a gavage probe #10 was shoved into the aorta. Approximately 20 ml of PBS were injected (~10 ml/min, room temperature) followed by 30 ml of 2% PFA in PBS. Brains were taken out and incubated for an additional 7h00 in the same perfusat. Vibratome was used to generate 100 microns brain free-floating sections that were used for immunofluorescence staining. Sections were first permeabilized and blocked using PBS-0.3% Triton X-100-10% donkey serum. Then, sections were incubated overnight with indicated primary antibodies diluted in PBS-5% donkey serum. Molecular probes secondary antibodies were used following manufacturer recommendations. Images were acquired using a Leica SP5 confocal microscope, Imaris software was used for image processing and 3D reconstruction.

These data illustrates the uptake of peripherally administered sFab-MAb31 and dFab-MAb31 by brain endothelial cells. MAb31, sFab-MAb31 and dFab-MAb31 were detected using a goat anti-human antibody coupled to Alexa 555. As shown in Fig 9, both sFab-MAb31 (Fig. 9A) and dFab-MAb31 (Fig. 9B) decorates the brain vasculature 15 min after injection with no difference in their distribution. 8h00 post-injection, sFab-MAb31 reaches the parenchyma and decorates amyloid plaques (Fig. 9C arrows) whereas dFab-MAb31 (Fig. 9D) stays within brain vasculature similarly to the 15min time point. No amyloid plaques in the parenchyma are detected with the dFab-MAb31.

Fig 10: To control the integrity of all constructs used in the study, staining of 18 months brain cryosections was done using MAb31 (Fig. 10A), sFab-MAb31 (Fig. 10B) or dFab-MAB31 (Fig. 10C). Results showed that all 3 constructs detected amyloid plaques in the brain of transgenic mice.

Fig 11-12: High resolution confocal microscopy shows that sFab (Fig. 11) and dFab-MAb31 (Fig. 12) do not decorate the luminal side of brain capillaries but are contained within vesicle-like structures crossing the luminal membrane of endothelial cells and within the endothelial cell cytosol. Arrows in Fig 11 and Fig 12 indicate vesicles containing sFab or dFab-MAb31 constructs on the abluminal side of endothelial cell nuclei. Altogether these data suggest that both sFab-MAb31 and dFab-MAb31 can enter endothelial cells but only sFab-MAb31 can cross the vasculature and reach amyloid plaques

The methods and compositions of the invention provide a way to drastically improve the part of the antibody that distributes into the CNS and thus more readily reach a therapeutic concentration in the CNS. The methods and compositions of the present invention are novel and significantly improve the efficiency of crossing through the different organelles within the BECs using an optimal and undisturbed intracellular route/sorting to reach the abluminal side.

**Example 8: Monovalent receptor binding mode crucial for crossing the BBB**

The anti-Aβ monoclonal antibody mAb31 is a very specific and potent Aβ plaque binder providing us with a powerful readout to quantify target engagement within brain parenchyma. We used the PS2APP double transgenic amyloidosis model to investigate the amount of brain exposure of the two Brain Shuttle constructs compared to the mAb31 parent antibody. The three variants were injected intravenously at 10 mg/kg and the degree of brain exposure was determined by quantifying the amount of antibody present at plaques 8 hours post injection. For the dFab construct no significant increase in plaque decoration was detected compared to mAb31 (Fig. 13A). However, for the sFab construct there was a massive increase in plaque decoration in comparison with the parent mAb31 antibody. Target engagement at the amyloid plaques was improved more than 50-fold for the sFab construct based on fluorescence intensity quantification using a labeled secondary antibody. Whereas the sFab construct showed extensive plaque decoration (Fig. 13D), the dFab was only detectable in the microvessels (Fig. 13C) indicating that the dFab construct targets and enters brain microvessels but fails to escape at the abluminal side. We investigated the target engagement capacity of the sFab construct at a low dose of 2.66 mg/kg and prolonged in vivo exposure time up to 7 days. Maximal plaque decoration was reached within 8 hours, followed by persistent plaque binding over at least one week after a single injection (Fig. 13E).

In a previous study, the parent mAb31 had been shown to reach maximal plaque binding 7 days after injection. Quantification of the staining in microvessel structures indicated that the localization of the sFab construct was very transient at the BBB, illustrating the relatively rapid rate at which the construct crosses the barrier. The representative plaque staining images for the parent antibody mAb31 at 2 mg/kg 7 day post injection (Fig. 13F) and equimolar concentration for the sFab construct (Fig. 13G) illustrate the increase in plaque binding one achieves with the sFab brain shuttle construct. The sFab construct shows only a minor colocalization with the lysosomal compartment, which likely reflects normal constitutive trafficking of the TfR to the lyso-some. Our in vitro studies also showed recycling and transcytosis of the sFab construct. Taken together, these findings suggest that the sFab construct does not interfere with the normal trafficking of the TfR. In contrast, the dFab construct shows strong colocalization with the lysosomal compartment but no transcytosis activity, neither in vitro nor in vivo.

**Example 9: Increased antibody delivery across BBB translates into enhanced in vivo potency**

In the next set of experiments we asked whether the significant increase in brain exposure using a monovalent binding mode improves in vivo potency of the anti-Aβ antibody in a long-term treatment study. We injected the sFab construct and the control parent antibody mAb31 weekly for three months. In a previous 5-months study, the therapeutic antibody mAb31 had been shown to reduce the plaque burden at 20 mg/kg. Based on the data shown in Figure 14 we selected two low doses to investigate if improved brain exposure would lead to enhanced in vivo potency. Target plaque binding at the end indicated that at both doses there was stronger target engagement with the sFab construct than the parent mAb31 antibody (Fig. 14 A - D).The degree of amyloidosis in the APPPS2 double transgenic mice was quantified at baseline, and following vehicle, low dose parent mAb31 and low dose sFab construct treatment. At these low doses no in vivo effect was detected with the parent monoclonal mAb31 (Fig. 14E), which was anticipated based on a previous long-term study over 5-months. In contrast, a significant reduction in plaque numbers both in cortex and hippocampus was observed with the 2.67 mg/kg low dose of the sFab construct. Even at the much lower dose of 0.53 mg/kg (Fig. 14E), a trend was seen in favor of the sFab construct especially in the cortex, although it did not reached statistical significance. A secondary analysis of plaque sizes revealed a more pronounced reduction of plaque numbers for small plaques, in agreement with the mode of action for mAb31. These data indicate that increased brain penetration, enabled by a monovalent mode of TfR binding, leads to a significant improvement in potency of a therapeutic antibody in a chronic animal model of Alzheimer's disease pathology.

**Example 10: Effector function of different antibody fusion proteins on TfR+ BaF3 cells in vitro (ADCC)**

Transferrin receptor expressing BaF3 cells (DSMZ, # CLPZ04004) (TfR+) were used as target cells for antibody-dependent cell toxicity (ADCC) experiments induced by different antibody-fusion molecules.

Briefly, $1\times10^4$ BaF3 cells were seeded in round bottom 96-wells and optionally co-cultured with human NK92 effector cells (high affinity CD16 clone 7A2F3; Roche GlycArt) at an effector/target ratio of 3:1 in the presence or absence of antibody fusion proteins. After four hours incubation (37°C, 5%CO2), cytotoxicity was assessed as measured by the release of lactate dehydrogenase (LDH) from dead/dying cells. For this cells were centrifuged for 5 min at 250xg and 50 μl supernatant was transferred to a flat bottom plate. 50 μl LDH reaction mix (Roche LDH reaction mix, cat. no. 11644793001; Roche Diagnostics GmbH) was added and the reaction was incubated for 20 min at 37°C, 5% $CO_2$. Subsequently, the absorbance was measured at a Tecan Sunrise Reader at 492/620nm wavelength.

All samples were tested in triplicates and the results calculated based the following controls:

- Only target cells (+ medium)

- Maximal LDH release: target cells + 3% Triton-X

- Spontaneous release: target cells + NK cells (E:T of 3:1)

% specific ADCC/lysis was calculated by the following term:

$$\% \text{ spec. ADCC} = \frac{\text{Sample} - \text{spontaneous release}}{\text{Maximal release - spontaneous release}} \times 100$$

Fig. 15: Antibody fusion with TfR scFab fragments fused to the Fc C-terminus do not induce ADCC. NK92-mediated killing of BA/F3 mouse erythroleukemia cells was measured by quantifying LDH release. Only fusion constructs with the TfR-binding Fab moiety in the "conventional" "N-terminal to Fc" orientation induce significant ADCC, while the brain shuttle constructs in reverse orientation are silent. Constructs: 8D3-IgG (full length 8D3 IgG), OA - 8D3 (single heavy chain of 8D3 IgG), mAb31 (antibody of Fig. 1A), mAb31-8D3 sFab (construct of Fig. 1B), mAb31-8D3-dFab (construct of Fig. 1C).

**Example 11: Epitope mapping of mTfR antibody 8D3**

The epitope mapping of monoclonal antibody 8D3 was carried out by means of a library of overlapping, immobilized peptide fragments (length: 15 amino acids, shift: 3 amino acids) corresponding to the sequence of the extracellular domain of murine Transferrin receptor 1 (90-763). For preparation of the peptide array the Intavis Cellu-Spots™ technology was employed. In this approach, peptides are synthesized with an automated synthesizer (Intavis MultiPep RS) on modified cellulose disks which are dissolved after synthesis. The solutions of the individual peptides that remain covalently linked to macromolecular cellulose are then spotted onto coated microscope slides. The CelluSpots™ synthesis was carried out stepwise utilizing 9-fluorenylmethoxycarbonyl (Fmoc) chemistry on amino-modified cellulose disks in a 384-well synthesis plate. In each coupling cycle, the corresponding amino acids were activated with a solution of DIC/HOBt in DMF. Between coupling steps, un-reacted amino groups were capped with a mixture of acetic anhydride, diisopropylethyl amine and 1-hydroxybenzotriazole. Upon completion of the synthesis, the cellulose disks were transferred to a 96-well plate and treated with a mixture of trifluoroacetic acid (TFA), dichloromethane, triisoproylsilane (TIS) and water for side chain deprotection. After removal of the cleavage solution, the cellulose bound peptides are dissolved with a mixture of TFA, TFMSA, TIS and water, precipitated with diisopropyl ether and re-suspended in DMSO. These peptide solutions were subsequently spotted onto Intavis CelluSpots™ slides using an Intavis slide spotting robot.

For epitope analysis, the prepared slides were washed with ethanol and then Tris-buffered saline (TBS; 50 mM Tris, 137 mM NaCl, 2.7 mM KCl, pH 8) before a blocking step was carried out for 16 h at 4°C with 5 mL 10x Western Blocking Reagent (Roche Applied Science), 2.5 g sucrose in TBS, 0.1% Tween 20. After washing (TBS + 0.1% Tween 20), the slides were incubated with a solution (1 μg/mL) of antibody 8D3 in TBS + 0.1% Tween 20 at ambient temperature for 2 h. After washing, the slides were incubated for detection with an anti-mouse secondary HRP-antibody (1:20000 in TBS-T) followed by incubation with chemiluminescence substrate luminol and visualized with a LumiImager (Roche Applied Science). ELISA-positive SPOTs were quantified and through assignment of the corresponding peptide sequences the antibody binding epitopes were identified.

Fig. 16: 8D3 binds to three distinct peptides in the extracellular domain of mouse transferrin receptor. Binding of antibody 8D3 to 15mer peptides overlapping by three amino acids was revealed by chemiluminescent detection of antibody incubated on a CelluSpot slide carrying immobilized mTfR peptides. Box: Peptides #373, 374 and 376 bound by 8D3.

Table 1: mTfR extracellular domain peptide sequences bound by 8D3 in peptide mapping experiment.

| Peptide ID | Peptide Sequence | Sequence Number |
|---|---|---|
| 373 | I-G-Q-N-M-V-T-I-V-Q-S-N-G-N-L | Seq. Id. No. 14 |
| 374 | N-M-V-T-I-V-Q-S-N-G-N-L-D-P-V | Seq. Id. No. 15 |
| 376 | Q-S-N-G-N-L-D-P-V-E-S-P-E-G-Y | Seq. Id. No. 16 |

Herein is described a group of biotherapeutic constructs against a blood brain barrier receptor, in particular the transferrin receptor (TfR), that can deliver therapeutics including antibodies, proteins, peptides and small molecules across the BBB at therapeutically relevant doses. Distribution of certain engineered biotherapeutic constructs changed from cerebrovascular space to parenchyma space within a few hours after injection, indicating that these particular constructs utilizing an optimal transport pathway through the BECs to allow significant amount of biotherapeutics to be transcytosed through BECs to reach the parenchyma. The degree of biotherapeutic constructs uptake into and distribution in the CNS was completely dependent on the monovalent binding mode to the blood brain barrier receptor, in particular TfR. When the TfR become dimerized by the binding of the biotherapeutic construct to the R/BBB no detectable level within the parenchyma space was detected. A single systemic dose of the single Fab anti-Abeta monoclonal construct engineered using the methodology of the invention not only resulted in significant antibody uptake in brain, but also dramatically increase the decoration of the anti-Abeta monoclonal binding to pathological amyloid plaques. However, using a double Fab binding construct against the R/BBB, no detectable levels within the CNS was detected. The facts and experiments depicted in this specification illustrate key contributing mechanisms behind increasing uptake of a biotherapeutics (such as antibodies) into the CNS using a monovalent binding mode against an R/BBB. First, a dual (or multimeric) anti-R/BBB binding mode limit brain uptake by quickly down-regulate the R/BBB on the cell surface on the lumen side, thus reducing the total amount anti-R/BBB that can be taken up into the vasculature which is the first step in efficient BBB crossing. Secondly, a dual (or multimeric) anti-R/BBB binding mode induces a distinct miss-sorting intracellularly in the BECs that prevent the construct to reach the abluminal side. Strikingly, monovalent binding to the R/BBB improves brain uptake and distribution, with a complete shift observed in localization from the vasculature to the amyloid plaques within the CNS. Second, the engineered monovalent binding mode of the biotherapeutic constructs for the R/BBB is securing the recycling of the R/BBB to the lumen side to allow uptake of additional fusion polypeptide construct and transport to the abluminal side and into the parenchyma. Third, the monovalent binding mode biotherapeutic construct is engineered at the C-terminal end of the Fc part of an IgG which preserve the original format for a therapeutic monoclonal antibody which in most cases are critical for in vivo efficacy. This can also be accomplished by linking to other part of an IgG described within this specification. This is advantageous because already developed IgG monoclonals with established preclinical and clinical efficacy can be incorporated in this transport system without compromising established function and efficacy. Furthermore, receptor mediated transport (RMT)-based monovalent targeting R/BBB technology opens the door for a wide range of potential therapeutics for CNS diseases. The invention provides methods of engineering BBB-penetrant therapeutics preserving existing IgGs formats with proven therapeutic activities that greatly improve transport across the BBB and CNS distribution of the therapeutic.

**Disclosed amino acid sequences**

| Amino acid sequence name | Sequence Identification Number (Seq. Id. No.) |
|---|---|
| Mab31 heavy chain - scFab (8D3) | 1 |
| Mab31 light chain | 2 |
| Knob Mab31 heavy chain - scFab (8D3) | 3 |
| Hole Mab31 heavy chain - scFab (8D3) | 4 |
| Mab 31 $V_H$ CDR1 | 5 |
| Mab 31 $V_H$ CDR2 | 6 |
| Mab 31 $V_H$ CDR3 | 7 |
| Mab 31 $V_L$ CDR1 | 8 |
| Mab 31 $V_L$ CDR 2 | 9 |
| Mab 31 $V_L$ CDR3 | 10 |
| Mab 31 $V_H$ | 11 |
| Mab 31 $V_L$ | 12 |
| Peptide linker $(G_4S)_6G_2$ | 13 |
| 8D3 epitope mapping peptide 373 | 14 |
| 8D3 epitope mapping peptide 374 | 15 |
| 8D3 epitope mapping peptide 376 | 16 |

(continued)

| Amino acid sequence name | Sequence Identification Number (Seq. Id. No.) |
|---|---|
| Peptide linker $(G_4S)_4$ | 17 |

**Claims**

1. A blood brain barrier shuttle comprising a full length IgG antibody directed to a brain target, a linker and one scFab directed to the transferrin receptor, wherein the scFab is coupled by the linker to the C-terminal end of the Fc part of one of the heavy chains of the full length IgG antibody

2. The blood brain barrier shuttle of claim 1, wherein the scFab recognizes an epitope in the transferrin receptor comprised within the amino acid sequence of Seq. Id. No. 14, 15 or 16.

3. The blood brain barrier shuttle of claim 1 or 2, wherein the full length IgG antibody directed to a brain target is a full length IgG antibody directed to Aβ.

4. The blood brain barrier shuttle of claim 3, wherein the full length IgG antibody directed to Aβ comprises (a) H-CDR1 comprising the amino acid sequence of Seq. Id. No. 5, (b) H-CDR2 comprising the amino acid sequence of Seq. Id. No. 6, (c) H-CDR3 comprising the amino acid sequence of Seq. Id. No. 7, (d) L-CDR1 comprising the amino acid sequence of Seq. Id. No. 8, (e) L-CDR2 comprising the amino acid sequence of Seq. Id. No. 9 and (f) L-CDR3 comprising the amino acid sequence of Seq. Id. No. 10.

5. The blood brain barrier shuttle of claim 4, wherein the full length IgG antibody directed to Aβ comprises a $V_H$ domain comprising the amino acid sequence of Seq. Id. No. 11 and a $V_L$ domain comprising the amino acid sequence of Seq. Id. No. 12.

6. The blood brain barrier shuttle of claim 1 or 2, wherein the full length IgG antibody directed to a brain target is a full length IgG antibody directed to phosphorylated Tau.

7. The blood brain barrier shuttle of claim 1 or 2, wherein the full length IgG antibody directed to a brain target is a full length IgG antibody directed to alpha synuclein.

8. The blood brain barrier shuttle of claims 1 to 7, wherein the linker is a peptide linker, preferably a peptide which is an amino acid sequence with a length of at least 20 amino acids, more preferably with a length of 25 to 50 amino acids.

**Patentansprüche**

1. Blut-Hirn-Schranken-Shuttle, umfassend einen IgG-Antikörper voller Länge, der gegen ein Hirnziel gerichtet ist, einen Linker und ein scFab, das gegen den Transferrinrezeptor gerichtet ist, wobei das scFab mithilfe des Linkers an das C-terminale Ende des Fc-Teils einer der schweren Ketten des IgG-Antikörpers voller Länge gekoppelt ist.

2. Blut-Hirn-Schranken-Shuttle nach Anspruch 1, wobei das scFab ein Epitop in dem Transferrinrezeptor erkennt, das in der Aminosäuresequenz von SEQ ID NO:14, 15 oder 16 enthalten ist.

3. Blut-Hirn-Schranken-Shuttle nach Anspruch 1 oder 2, wobei der IgG-Antikörper voller Länge, der gegen ein Hirnziel gerichtet ist, ein IgG-Antikörper voller Länge ist, der gegen Aβ gerichtet ist.

4. Blut-Hirn-Schranken-Shuttle nach Anspruch 3, wobei der IgG-Antikörper voller Länge, der gegen Aβ gerichtet ist, (a) H-CDR1, umfassend die Aminosäuresequenz von SEQ ID NO:5, (b) H-CDR2, umfassend die Aminosäuresequenz von SEQ ID NO:6, (c) H-CDR3, umfassend die Aminosäuresequenz von SEQ ID NO:7, (d) L-CDR1, umfassend die Aminosäuresequenz von SEQ ID NO:8, (e) L-CDR2, umfassend die Aminosäuresequenz von SEQ ID NO:9, und (f) L-CDR3, umfassend die Aminosäuresequenz von SEQ ID NO:10, umfasst.

5. Blut-Hirn-Schranken-Shuttle nach Anspruch 4, wobei der IgG-Antikörper voller Länge, der gegen Aβ gerichtet ist, eine $V_H$-Domäne, umfassend die Aminosäuresequenz von SEQ ID NO:11, und eine $V_L$-Domäne, umfassend die Aminosäuresequenz von SEQ ID NO: 12, umfasst.

6. Blut-Hirn-Schranken-Shuttle nach Anspruch 1 oder 2, wobei der IgG-Antikörper voller Länge, der gegen ein Hirnziel gerichtet ist, ein IgG-Antikörper voller Länge ist, der gegen phosphoryliertes Tau gerichtet ist.

7. Blut-Hirn-Schranken-Shuttle nach Anspruch 1 oder 2, wobei der IgG-Antikörper voller Länge, der gegen ein Hirnziel gerichtet ist, ein IgG-Antikörper voller Länge ist, der gegen Alpha-Synuclein gerichtet ist.

8. Blut-Hirn-Schranken-Shuttle nach den Ansprüchen 1 bis 7, wobei der Linker ein Peptid-Linker, vorzugsweise ein Peptid, das eine Aminosäuresequenz mit einer Länge von mindestens 20 Aminosäuren, stärker bevorzugt mit einer Länge von 25 bis 50 Aminosäuren, ist.

**Revendications**

1. Navette de la barrière hémato-encéphalique comprenant un anticorps IgG pleine longueur dirigé contre une cible cérébrale, un lieur et un scFab dirigé contre le récepteur de la transferrine, dans laquelle le scFab est couplé par le lieur à l'extrémité C-terminale de la partie Fc de l'une des chaînes lourdes de l'anticorps IgG pleine longueur.

2. Navette de la barrière hémato-encéphalique selon la revendication 1, dans laquelle le scFab reconnait un épitope dans le récepteur de la transferrine compris au sein de la séquence d'acides aminés de Seq. Id. No. 14, 15 ou 16.

3. Navette de la barrière hémato-encéphalique selon la revendication 1 ou 2, dans laquelle l'anticorps IgG pleine longueur dirigé contre une cible cérébrale est un anticorps IgG pleine longueur dirigé contre A$\beta$.

4. Navette de la barrière hémato-encéphalique selon la revendication 3, dans laquelle l'anticorps IgG pleine longueur dirigé contre A$\beta$ comprend (a) une H-CDR1 comprenant la séquence d'acides aminés de Seq. Id. No. 5, (b) une H-CDR2 comprenant la séquence d'acides aminés de Seq. Id. No. 6, (c) une H-CDR3 comprenant la séquence d'acides aminés de Seq. Id. No. 7, (d) une L-CDR1 comprenant la séquence d'acides aminés de Seq. Id. No. 8, (e) une L-CDR2 comprenant la séquence d'acides aminés de Seq. Id. No. 9 et (f) une L-CDR3 comprenant la séquence d'acides aminés de Seq. Id. No. 10.

5. Navette de la barrière hémato-encéphalique selon la revendication 4, dans laquelle l'anticorps IgG pleine longueur dirigé contre A$\beta$ comprend un domaine $V_H$ comprenant la séquence d'acides aminés de Seq. Id. No. 11 et un domaine $V_L$ comprenant la séquence d'acides aminés de Seq. Id. No. 12.

6. Navette de la barrière hémato-encéphalique selon la revendication 1 ou 2, dans laquelle l'anticorps IgG pleine longueur dirigé contre une cible cérébrale est un anticorps IgG pleine longueur dirigé contre la Tau phosphorylée.

7. Navette de la barrière hémato-encéphalique selon la revendication 1 ou 2, dans laquelle l'anticorps IgG pleine longueur dirigé contre une cible cérébrale est un anticorps IgG pleine longueur dirigé contre l'alpha-synucléine.

8. Navette de la barrière hémato-encéphalique selon les revendications 1 à 7, dans laquelle le lieur est un lieur peptidique, de préférence un peptide qui est une séquence d'acides aminés ayant une longueur d'au moins 20 acides aminés, plus préférablement ayant une longueur de 25 à 50 acides aminés.

Fig. 1A

Fig. 1B

Fig. 1C

Fig. 2

**Fig. 3**

EP 3 315 514 B1

Fig. 4C

dFab-mAb31

Fig. 4B

sFab-mAb31

Fig. 4A

mAb31

Fig. 5

# Quantification:
**555 nm fluorescence intensity. 8h post dose**

EP 3 315 514 B1

**Fig. 6**

Fig. 7

Fig. 8A

Fig. 8B

EP 3 315 514 B1

Fig. 9A

Fig. 9B

Fig. 9C

Fig. 9D

**Fig. 10A**  **Fig. 10B**  **Fig. 10C**  **Fig. 10D**

**MAb31**  **sFab-MAb31**  **dFab-MAb31**  **Control**

Fig. 11

Fig. 12

**Fig. 13A**

**Fig. 13B**       mAb31

**Fig. 13C**       dFab

**Fig. 13D**       sFab

**Fig. 13E**

**Fig. 13F**

**mAb31**

**Fig. 13G**

**sFab**

**Fig. 14A**

**Low dose mAb31**

**Fig. 14B**

**Mid dose mAb31**

**Fig. 14C**

**Low dose sFab**

**Fig. 14D**

**Mid dose sFab**

**Fig. 14E**

Cortex

Hippocampus

EP 3 315 514 B1

**Fig. 15**

EP 3 315 514 B1

Fig. 16

Peptides #373, 374 and 376

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012075037 A
- US 4816567 A
- US 5693780 A
- US 5591669 A
- US 5589369 A
- US 5545807 A
- US 5565332 A
- US 5573905 A
- US 5567610 A
- US 5229275 A
- US 20020004587 A1, Miller
- WO 0042072 A

- WO 9951642 A, Iduosogie
- US 20030157108 A, Presta, L.
- US 20040093621 A
- WO 2003011878 A, Jean-Mairet
- US 6602684 B, Umana
- WO 199730087 A, Patel
- WO 199858964 A, Raju, S
- WO 199922764 A, Raju, S
- US 20050123546 A, Umana
- US 5208020 A
- US 3773919 A

### Non-patent literature cited in the description

- **FELGENHAUER**. *Klin. Wschr.*, 1974, vol. 52, 1158-1164
- **CARTER P.** ; **RIDGWAY J.B.B.** ; **PRESTA L.G.** *Immunotechnology*, February 1996, vol. 2 (1), 73-73
- **SCHNEIDER et al.** *Nature*, 1984, vol. 311, 675-678
- **HUST M. et al.** *BMC Biotechnol.*, 08 March 2007, vol. 7, 14
- **KOHLER et al.** *Nature*, 1975, vol. 256, 495
- **CLACKSON et al.** *Nature*, 1991, vol. 352, 624-628
- **MARKS et al.** *J. Mol. Biol.*, 1991, vol. 222, 581-597
- **MORRISON et al.** *Proc. Natl. Acad. Sci. USA*, 1984, vol. 81, 6851-6855
- **JONES et al.** *Nature*, 1986, vol. 321, 522-525
- **RIECHMANN et al.** *Nature*, 1988, vol. 332, 323-329
- **PRESTA**. *Curr. Op. Struct. Biol*, 1992, vol. 2, 593-596
- **JAKOBOVITS et al.** *Proc. Natl Acad. Sci. USA*, 1993, vol. 90, 2551
- **JAKOBOVITS et al.** *Nature*, 1993, vol. 362, 255-258
- **BRUGGERMANN et al.** *Year in Immuno*, 1993, vol. 7, 33

- **MCCAFFERTY et al.** *Nature*, 1990, vol. 348, 552-553
- **JOHNSON et al.** *Current Opinion in Structural Biology*, 1993, vol. 3, 564-571
- **GRIFFITH et al.** *EMBO J.*, 1993, vol. 12, 725-734
- **KONTERMANN**. Dual targeting strategies with bispecific antibodies. *MABS*, March 2012, vol. 4 (2), 182-197
- **KABAT et al.** Sequences of Proteins of Immunological Interest. Public Health Service, National Institutes of Health, 1991
- **CHOTHIA** ; **LESK**. *J. Mol. Biol.*, 1987, vol. 196, 901-917
- **CHARI et al.** *Cancer Res.*, 1992, vol. 52, 127-131
- **ZATSEPIN et al.** *Russ. Chem. Rev.*, 2005, vol. 74, 77-95
- **FLATMAN et al.** *J. Chromatogr. B*, 2007, vol. 848, 79-87
- Remington's Pharmaceutical Sciences. 1980
- Remington's Pharmaceutical Sciences. 1980
- **BOADO, R.J. ZHANG** ; **Y. WANG, Y** ; **PARDRIDGE, W.M.** *Biotechnology and Bioengineering*, 2009, vol. 102, 1251-1258
- **BOADO, R.J. ZHANG** ; **Y. WANG, Y** ; **PARDRIDGE, W.M.** *Biotechnology and Bioengineering*, 2009, vol. 102, 1251-1258